(19) Europäisches Patentamt

European Patent Office

Office européen des brevets

(11) **EP 1 289 919 B1**

(12) **EUROPÄISCHE PATENTSCHRIFT**

(45) Veröffentlichungstag und Bekanntmachung des Hinweises auf die Patenterteilung:
**29.11.2006   Patentblatt 2006/48**

(51) Int Cl.:
*C07C 45/33* *(2006.01)*   *C07C 45/35* *(2006.01)*
*C07C 47/22* *(2006.01)*   *C07C 51/215* *(2006.01)*
*C07C 51/25* *(2006.01)*   *C07C 57/04* *(2006.01)*
*C07C 5/48* *(2006.01)*    *C07C 11/06* *(2006.01)*
*C07C 7/11* *(2006.01)*

(21) Anmeldenummer: 01956472.3

(22) Anmeldetag: **13.06.2001**

(86) Internationale Anmeldenummer:
**PCT/EP2001/006708**

(87) Internationale Veröffentlichungsnummer:
**WO 2001/096271 (20.12.2001 Gazette 2001/51)**

(54) **VERFAHREN ZUR HERSTELLUNG VON ACROLEIN UND/ODER ACRYLSÄURE**

METHOD FOR PRODUCING ACROLEIN AND/OR ACRYLIC ACID

PROCEDE DE PRODUCTION D'ACROLEINE ET/OU D'ACIDE ACRYLIQUE

(84) Benannte Vertragsstaaten:
**AT BE CH CY DE DK ES FI FR GB GR IE IT LI LU MC NL PT SE TR**

(30) Priorität: **14.06.2000   DE 10028582**
**20.06.2000   DE 10029338**
**07.07.2000   DE 10033121**
**18.07.2000   DE 10034825**
**20.09.2000   DE 10046672**
**17.10.2000   DE 10051419**
**17.04.2001   DE 10118814**
**23.04.2001   DE 10119933**
**03.05.2001   DE 10121592**
**07.05.2001   DE 10122027**

(43) Veröffentlichungstag der Anmeldung:
**12.03.2003   Patentblatt 2003/11**

(60) Teilanmeldung:
**05025498.6 / 1 642 879**

(73) Patentinhaber: **BASF Aktiengesellschaft**
**67056 Ludwigshafen (DE)**

(72) Erfinder:
• **MACHHAMMER, Otto**
**68163 Mannheim (DE)**
• **ADAMI, Christoph**
**69469 Weinheim (DE)**
• **HECHLER, Claus**
**67063 Ludwigshafen (DE)**
• **ZEHNER, Peter**
**67071 Ludwigshafen (DE)**

(74) Vertreter: **Isenbruck, Günter et al**
**Isenbruck, Bösl, Hörschler, Wichmann, Huhn**
**Patentanwälte**
**Theodor-Heuss-Anlage 12**
**68165 Mannheim (DE)**

(56) Entgegenhaltungen:
**EP-A- 0 117 146        EP-A- 0 608 838**
**EP-A- 0 962 253        FR-A- 2 754 817**
**GB-A- 1 378 178        GB-A- 2 118 939**

**Beschreibung**

Die vorliegende Erfindung betrifft ein Verfahren zur Herstellung von Acrolein und/oder Acrylsäure aus Propen oder Propan und Propen.

Acrolein und Acrylsäure sind bedeutende Grundchemikalien. So findet Acrylsäure unter anderem Verwendung als Monomeres zur Herstellung von Polymerisaten, die beispielsweise in disperser Verteilung in wäßrigem Medium befindlich als Bindemittel angewendet werden. In Abhängigkeit vom Verwendungszweck des Polymerisats kann vor der Polymerisation noch eine Veresterung der Acrylsäure stattfinden. Acrolein ist ein bedeutendes Zwischenprodukt, beispielsweise für die Herstellung von Glutardialdehyd, Methionin, Folsäure und Acrylsäure.

Bekannte Verfahren zur Herstellung von Acrolein und/oder Acrylsäure gehen von Propan und/oder Propen aus. Aus der DE-A 33 13 573 und EP-A-0 117 146 ist ein Verfahren zur Umsetzung von Propan zu Acrolein und/oder Acrylsäure bekannt. Dabei wird ein Zweibeziehungsweise Dreistufenverfahren beschrieben, bei dem in einer ersten Stufe das Propan zu Propen dehydriert und in einer zweiten Stufe das Propen zu Acrolein oxidiert wird. Wesentliches Merkmal ist hierbei, dass zwischen den beiden Stufen keine Abtrennung des Propans von bei der Dehydrierung gebildeten Nebenkomponenten, wie zum Beispiel molekularem Wasserstoff, vorgenommen wird. Die Oxidation wird unter Bedingungen durchgeführt, unter denen keine merkliche Oxidation des Wasserstoffs stattfindet. In einer dritten Stufe kann das Acrolein dann zu Acrylsäure oxidiert werden. Es wird auch die Möglichkeit vorgesehen, nicht umgesetztes Propan und Propen aus der zweiten oder dritten Stufe durch Absorption abzutrennen und nach Freisetzung vom Absorptionsmittel wieder der ersten Stufe (Dehydrierungsstufe) zurückzuführen.

Die japanische Patentanmeldung JP-A 10-36311 offenbart ein Verfahren zur Herstellung von α,β-ungesättigten Carbonsäuren, wie Acrylsäure, durch Gasphasen-katalytische Oxidation des Propans in Anwesenheit eines Komposit-Metalloxid-Katalysators, wobei zur Erzielung hoher Ausbeuten in dem Ausgangsgemisch das Verhältnis von Propan zu Sauerstoff und gegebenenfalls Verdünnungsgas in einem definierten Bereich gehalten wird, ebenso wie der Umsatz bei einem bestimmten Wert gehalten wird. Nicht umgesetztes Propan kann nach der Oxidation durch einen selektiven Separator abgetrennt werden, der Druck-Wechsel-Adsorptionseinheiten (*Pressure-Swing-Adsorption*) umfasst, und anschließend der Gasphasen-katalytischen Oxidation wieder zurückgeführt werden.

GB 1378-178 offenbart ein Verfahren, bei dem ein in einem Oxidationsprozess nicht umgesetzter Kohlenwasserstoff in ein Absorptionsmittel absorbiert und das Absorptionsmittel mit einem Strippmedium gestrippt wird. Dabei wird dem Strippmedium der zu gewinnende Kohlenwasserstoff in einer solchen Menge zugesetzt, dass das Gemisch ausserhalb der Entzündungsgrenzen liegt.

EP-A 0 731 077 offenbart ein Verfahren zur Herstellung von Acrolein, Acrylsäure oder deren Gemisch aus Propan, bei dem in einer ersten Stufe Propan partiell zu Propylen dehydriert wird, das dabei resultierende Produktgasgemisch nach Abtrennung von Wasserstoff und Wasserdampf zur Beschickung eines Oxidationsreaktors verwendet wird, in selbigem das bei der Dehydrierung gebildete Propylen mit molekularem Sauerstoff im Beisein von nicht dehydriertem Propan als inertem Verdünnungsgas zu Acrolein, Acrylsäure oder deren Gemisch partiell oxidiert wird und das im Produktgasgemisch der partiellen Oxidation enthaltene Propan anschließend in die Dehydrierstufe A zurückführt wird.

Die Aufgabe der vorliegenden Erfindung bestand darin, ein Verfahren zur Gasphasen-katalytischen Herstellung von Acrolein und/oder Acrylsäure aus Propan und/oder Propen zu schaffen, das wirtschaftlich ist und bei dem der eingesetzte Katalysator möglichst lange ohne Regenerierung einsetzbar ist.

Diese Aufgabe wird erfindungsgemäß gelöst durch ein Verfahren zur Herstellung von Acrolein und/oder Acrylsäure aus Propan wobei das Verfahren folgende Schritte umfaßt:

(a) homogene und/oder heterogen katalytische Dehydrierung von Propan zu Propen unter Erhalt eines Propen oder eines Propen und Propan enthaltenden Gasgemisches A,
(b) Abtrennung von Propen oder Propen und Propan aus dem Gasgemisch A durch Absorption in ein Absorptionsmittel,
(c) Abtrennung des Propens oder des Propens und Propans von dem Absorptionsmittel unter Erhalt eines Propen oder eines Propen und Propan enthaltenden Gases B und
(d) Verwendung des in Stufe (c) erhaltenen Gases B für eine Oxidation von Propen zu Acrolein und/oder Acrylsäure,

wobei zwischen Schritt (c) und (d) keine heterogen katalysierte Dehydrierung von Propan ohne Zufuhr von Sauerstoff durchgeführt wird,
wobei die Dehydrierung oxidativ erfolgt und Schritt (d) direkt nach Schritt (c) durchgeführt wird.

Bevorzugte Ausführungsformen der Erfindung ergeben sich aus der nachfolgenden Beschreibung sowie den Figuren.

Da das Propen oder Propan und Propen vor der Oxidation einer Absorption unterzogen wird, werden in der Regel Reste von Absorptionsmittel in dem Gas B vorliegen. Überraschenderweise wurde nun gefunden, dass trotzdem bei der Oxidation keine Probleme auftreten. So wurde beim Oxidationskatalysator keine wesentliche Abnahme der Aktivität beobachtet und der Oxidationskatalysator konnte ohne Regenerierung über eine lange Betriebsdauer eingesetzt werden. Auch wurden keine Probleme durch eine gegebenenfalls zu erwartende Mitoxidation von Resten von Absorptionsmittel in der Oxidationsstufe beobachtet.

Bei dem Verfahren gemäß DE-A 33 13 573 wird durch Absorption rückgewonnenes beziehungsweise abgetrenntes Propan und Propen in die heterogen katalysierte Dehydrierung des Propans zurückgeführt. Bei der heterogen katalysierten Propan-Dehydrierung kann der Katalysator, beispielsweise durch Verkokung, deaktiviert werden. Deshalb werden solche Dehydrierungskatalysatoren häufig regeneriert. Daher stellt in der Propandehydrierung mit dem Gasstrom mitgeführtes Absorptionsmittel kein Problem dar, da es zusammen mit der Verkokung abgebrannt werden kann. Hingegen werden die in der Oxidation zu Acrolein und/oder Acrylsäure eingesetzten Katalysatoren üblicherweise nicht so oft regeneriert, weshalb der zusätzliche Regenerierungsaufwand, der dadurch zustande kommt, daß das Einsatzgas Absorptionsmittel enthält, größer ist als im Fall der Dehydrierung. Das erfindungsgemäße Verfahren bietet den Vorteil, daß der Oxidationskatalysator über lange Zeit ohne Regenerierung eingesetzt werden kann.

Von dem Verfahren gemäß DE-A 33 13 573 unterscheidet sich das erfindungsgemäße Verfahren darin, daß das durch Absorption abgetrennte Propen oder Propan und Propen in eine Oxidationsstufe geführt wird. Anders als in dem Verfahren gemäß DE-A 33 13 573 findet erfindungsgemäß zwischen der Abtrennung des Propens oder Propans und Propens von dem Absorptionsmittel und der Oxidation zu Acrolein und/oder Acrylsäure keine heterogen katalysierte Dehydrierung von Propan ohne Sauerstoffzufuhr statt.

Im Rahmen der vorliegenden Erfindung kann es sich bei dem Gas B auch um ein Gasgemisch handeln.

In Schritt (b) können Gasgemische A mit beliebigen Mengen an Propen oder Propan und Propen eingesetzt werden. Vorzugsweise enthält das Gasgemisch A Propan und Propen im molaren Verhältnis von 0 : 100 bis 100 : 0, insbesondere 10 : 90 bis 90 : 10, häufig 80:20 bis 40:60.

Vorzugsweise enthält das Gasgemisch A wenigstens eine weitere, von Propan und/oder Propen verschiedene Komponente, die keinen besonderen Beschränkungen unterliegt. In der Regel hängen die weiteren Komponenten davon ab, woher das Gasgemisch stammt. Insbesondere handelt es sich hierbei um wenigstens eine Komponente ausgewählt aus Stickstoff, Wasserstoff, Kohlenoxiden wie Kohlenmonoxid oder Kohlendioxid, weiteren aus einer Dehydrierung von Propan stammenden Nebenkomponenten, aus einer Gasphasenoxidation von Propen zu Acrolein und/oder Acrylsäure stammenden Nebenkomponenten oder aus einer Oxidation von Propan zu Acrolein und/oder Acrylsäure stammenden Nebenkomponenten. Häufig liegt als weitere Komponente wenigstens Wasserstoff, Stickstoff, Kohlenoxid oder ein Gemisch von diesen vor.

Als Absorptionsmittel in Schritt (b) eignen sich grundsätzlich alle Absorptionsmittel, die in der Lage sind, Propan und Propen zu absorbieren. Bei dem Absorptionsmittel handelt es sich vorzugsweise um ein organisches Lösungsmittel, das vorzugsweise hydrophob und/oder hochsiedend ist. Vorteilhafterweise hat dieses Lösungsmittel einen Siedepunkt (bei einem Normaldruck von 1 atm) von mindestens 120°C, bevorzugt von mindestens 180 °C, vorzugsweise von 200 bis 350°C, insbesondere von 250 bis 300°C, stärker bevorzugt von 260 bis 290°C. Zweckmäßigerweise liegt der Flammpunkt (bei einem Normaldruck von 1 atm) über 110°C. Allgemein eignen sich als Absorptionsmittel relativ unpolare organische Lösungsmittel, wie zum Beispiel aliphatische Kohlenwasserstoffe, die vorzugsweise keine nach außen wirkende polare Gruppe enthalten, aber auch aromatische Kohlenwasserstoffe. Allgemein ist es erwünscht, dass das Absorptionsmittel einen möglichst hohen Siedepunkt bei gleichzeitig möglichst hoher Löslichkeit für Propan und/oder Propen hat. Beispielhaft als Absorptionsmittel genannt seien aliphatische Kohlenwasserstoffe, zum Beispiel $C_8$-$C_{20}$-Alkane oder -Alkene, oder aromatische Kohlenwasserstoffe, zum Beispiel Mittelölfraktionen aus der Paraffindestillation oder Ether mit sperrigen Gruppen am O-Atom, oder Gemische davon, wobei diesen ein polares Lösungsmittel, wie zum Beispiel das in der DE-A 43 08 087 offenbarte 1,2-Dimethylphthalat zugesetzt sein kann. Weiterhin eignen sich Ester der Benzoesäure und Phthalsäure mit geradkettigen, 1 bis 8 Kohlenstoffatome enthaltenden Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkene, zum Beispiel 4-Methyl-4'-benzyl-diphenylmethan und dessen Isomere 2-Methyl-2'-benzyl-diphenyl-methan, 2-Methyl-4'-benzyldiphenylmethan und 4-Methyl-2'-benzyl-diphenylmethan und Gemische solcher Isomerer. Ein geeignetes Absorptionsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, beispielsweise das im Handel erhältliche Diphyl. Häufig enthält dieses Lösungsmittelgemisch ein Lösungsmittel wie Dimethylphthalat in einer Menge von 0,1 bis 25 Gew.-%, bezogen auf das gesamte Lösungsmittelgemisch, zugesetzt. Besonders geeignete Absorptionsmittel sind auch Octane, Nonane, Decane, Undecane, Dodecane, Tridecane, Tetradecane, Pentadecane, Hexadecane, Heptadecane und Octadecane, wobei sich insbesondere Tetradecane als besonders geeignet erwiesen haben. Günstig ist es, wenn das verwendete Absorptionsmittel einerseits den oben genannten Siedepunkt erfüllt, andererseits gleichzeitig aber ein nicht zu hohes Molekulargewicht aufweist. Mit Vorteil beträgt das Molekulargewicht des Absorptionsmittels ≤ 300 g/mol. Geeignet sind auch die in DE-A 33 13 573 beschriebenen Paraffinöle mit 8 bis 10 Kohlenstoffatomen. Beispiele für geeignete Handelsprodukte sind von der Firma Haltermann vertriebene Produkte wie Halpasole i, wie Halpasol 250/340 i und Halpasol 250/275 i, sowie Druckfarbenöle unter den Bezeichnungen PKWF und Printosol.

Die Durchführung der Absorption unterliegt keinen besonderen Beschränkungen. Es können alle dem Fachmann gängigen Verfahren und Bedingungen eingesetzt werden. Vorzugsweise wird das Gasgemisch bei einem Druck von 1 bis 50 bar, bevorzugt 2 bis 20 bar, stärker bevorzugt 5 bis 10 bar, und einer Temperatur von 0 bis 100°C, insbesondere von 30 bis 50°C, mit dem Absorptionsmittel in Kontakt gebracht. Die Absorption kann sowohl in Kolonnen als auch in Quenchapparaten vorgenommen werden. Dabei kann im Gleichstrom oder im Gegenstrom gearbeitet werden. Geeignete Absorptionskolonnen sind zum Beispiel Bodenkolonnen (mit Glocken- und/oder Siebböden), Kolonnen mit strukturierten Packungen (zum Beispiel Blechpackungen mit einer spezifischen Oberfläche von 100 bis 500 $m^2/m^3$, zum Beispiel Mellapak® 250 Y) und Füllkörperkolonnen (zum Beispiel mit Raschig-Füllkörpern gefüllte). Es können aber auch Riesel- und Sprühtürme, Graphitblockabsorber, Oberflächenabsorber wie Dickschicht- und Dünnschichtabsorber sowie Tellerwäscher, Kreuzschleierwäscher und Rotationswäscher verwendet werden. Zudem kann es günstig sein, die Absorption in einer Blasensäule mit und ohne Einbauten stattfinden zu lassen.

Die Abtrennung des Propens oder Propans und Propens von dem Absorptionsmittel kann durch Strippung, Entspannungsverdampfung (*Flashen*) und/oder Destillation erfolgen.

Die Abtrennung des Propens oder Propans und Propens von dem Absorptionsmittel in Schritt (c) erfolgt vorzugsweise durch Strippen beziehungsweise Desorption mit einem bezüglich des erfindungsgemäßen Schrittes (d) sich inert verhaltenden Gas und/oder molekularem Sauerstoff (zum Beispiel Luft). Das Strippen kann hierbei in üblicher Weise über eine Druck- und/oder Temperaturänderung durchgeführt werden, vorzugsweise bei einem Druck von 0,1 bis 10 bar, insbesondere 1 bis 5 bar, stärker bevorzugt 1 bis 2 bar, und einer Temperatur von 0 bis 200°C, insbesondere 20 bis 100°C, stärker bevorzugt 30 bis 50°C. Ein anderes für die Strippung geeignetes Gas ist beispielsweise Wasserdampf, bevorzugt sind jedoch insbesondere Sauerstoff-/Stickstoff-Mischungen, beispielsweise Luft. Bei der Verwendung von Luft beziehungsweise Sauerstoff-/Stickstoff-Mischungen, bei denen der Sauerstoffgehalt über 10 Vol-% liegt, kann es sinnvoll sein, vor oder während des Stripprozesses ein Gas zuzusetzen, das den Explosionsbereich reduziert. Besonders geeignet dafür sind Gase mit einer spezifischen Wärmekapazität >29 J/mol·K bei 20°C, wie zum Beispiel Methan, Ethan, Propan, Butan, Pentan, Hexan, Benzol, Methanol, Ethanol, sowie Ammoniak, Kohlendioxid, und Wasser. Besonders geeignet für die Strippung sind auch Blasensäulen mit und ohne Einbauten.

Die Abtrennung des Propens oder Propans und Propens von dem Absorptionsmittel kann auch über eine Destillation erfolgen, wobei die dem Fachmann geläufigen Kolonnen mit Packungen, Füllkörpern oder entsprechenden Einbauten verwendet werden können. Bevorzugte Bedingungen bei der Destillation sind ein Druck von 0,01 bis 5 bar, insbesondere 0,1 bis 3 bar, stärker bevorzugt 1 bis 2 bar, und eine Temperatur (im Sumpf) von 50 bis 300°C, insbesondere 150 bis 250°C.

Schritt (d) wird direkt nach Schritt (c) durchgeführt, das heißt, ohne Verfahrensschritte dazwischen oder Zwischenstufen.

Die in Schritt (d) durchgeführte Oxidation von Propen zu Acrolein und/oder Acrylsäure kann gemäß allen dem Fachmann bekannten Verfahren durchgeführt werden, die keinen Beschränkungen unterliegen. In Schritt (d) kann eine ein- oder zweistufige Oxidation von Propen zu Acrolein und/oder Acrylsäure durchgeführt werden. Die Oxidation wird zweckmäßigerweise als selektive heterogen katalysierte Gasphasen-Oxidation mit molekularem Sauerstoff zu einem Acrolein und/oder Acrylsäure enthaltenden Produktgasgemisch durchgeführt. Bei Bedarf wird das der Oxidation zugeführte Propen oder Propan und Propen vorab durch indirekten Wärmetausch auf die für die Oxidationsreaktion erforderliche Reaktionstemperatur gebracht.

Prinzipiell läuft die heterogen katalysierte Gasphasen-Oxidation von Propen zu Acrylsäure mit molekularem Sauerstoff in zwei längs der Reaktionskoordinate aufeinanderfolgenden Schritten ab, von denen der erste zum Acrolein und der zweite vom Acrolein zur Acrylsäure führt. Dieser Reaktionsablauf in zwei zeitlich aufeinanderfolgenden Schritten eröffnet in an sich bekannter Weise die Möglichkeit, den Schritt (d) des erfindungsgemäßen Verfahrens in zwei hintereinander angeordneten Oxidationszonen auszuführen, wobei in jeder der beiden Oxidationszonen der zu verwendende oxidische Katalysator in optimierender Weise angepasst werden kann. So wird für die erste Oxidationszone (Propen → Acrolein) in der Regel ein Katalysator auf der Basis von die Elementkombination Mo-Bi-Fe enthaltenden Multimetalloxiden bevorzugt, während für die zweite Oxidationszone (Acrolein → Acrylsäure) normalerweise Katalysatoren auf der Basis von die Elementkombination Mo-V enthaltenden Multimetalloxiden bevorzugt werden. Entsprechende Multimetalloxidkatalysatoren für die beiden Oxidationszonen sind vielfach vorbeschrieben und dem Fachmann wohlbekannt. Beispielsweise verweist die EP-A-0 253 409 auf Seite 5 auf entsprechende US-Patente. Günstige Katalysatoren für die beiden Oxidationszonen offenbaren auch die DE-A 44 31 957 und die DE-A 44 31 949. Dieses gilt insbesondere für jene der allgemeinen Formel I in den beiden vorgenannten Schriften. In der Regel wird das Produktgemisch aus der ersten Oxidationszone ohne Zwischenbehandlung in die zweite Oxidationszone überführt.

Die einfachste Realisierungsform der beiden Oxidationszonen bildet daher ein Rohrbündelreaktor, innerhalb dessen sich die Katalysatorbeschickung längs der einzelnen Kontaktrohre mit Beendigung des ersten Reaktionsschrittes entsprechend ändert (derartige Oxidationen lehren zum Beispiel EP-A-0 911 313, EP-A-0 979 813, EP-A-0 990 636 und DE-A 28 30 765). Gegebenenfalls wird dabei die Beschickung der Kontaktrohre mit Katalysator durch eine Inertschüttung unterbrochen.

Vorzugsweise werden die beiden Oxidationszonen jedoch in Form zweier hintereinander geschalteter Rohrbündelsysteme realisiert. Diese können sich in einem Reaktor befinden, wobei der Übergang von einem Rohrbündel zu einem anderen Rohrbündel von einer nicht im Kontaktrohr untergebrachten (zweckmäßigerweise begehbaren) Schüttung aus Inertmaterial gebildet werden. Während die Kontaktrohre in der Regel von einem Wärmeträger umspült werden, erreicht dieser eine wie vorstehend beschrieben angebrachte Inertschüttung nicht. Mit Vorteil werden daher die beiden Kontaktrohrbündel in räumlich voneinander getrennten Reaktoren untergebracht. In der Regel befindet sich dabei zwischen den beiden Rohrbündelreaktoren ein Zwischenkühler, um eine gegebenenfalls erfolgende Acroleinnachverbrennung im Produktgasgemisch, das die erste Oxidationszone verläßt, zu mindern. Anstelle von Rohrbündelreaktoren können auch Plattenwärmetauscherreaktoren mit Salz- und/oder Siedekühlung, wie zum Beispiel in der DE-A 199 29 487 und der DE-A 199 52 964 beschrieben, eingesetzt werden.

Die Reaktionstemperatur in der ersten Oxidationszone liegt in der Regel bei 300 bis 450°C, bevorzugt bei 320 bis 390°C. Die Reaktionstemperatur in der zweiten Oxidationszone liegt in der Regel bei 200 bis 300°C, häufig bei 220 bis 290°C. Der Reaktionsdruck beträgt in beiden Oxidationszonen zweckmäßig 0,5 bis 5, vorteilhaft 1 bis 3 atm. Die Gasbelastung (Nl/l·h) der Oxidationskatalysatoren mit Reaktionsgas beträgt in beiden Oxidationszonen häufig 1500 bis 2500 h$^{-1}$ beziehungsweise bis 4000 h$^{-1}$. Die Propenbelastung (Nl/l·h) beträgt häufig 50 bis 300 h$^{-1}$, insbesondere 100 bis 200 h$^{-1}$.

Prinzipiell können die beiden Oxidationszonen so gestaltet werden, wie es zum Beispiel in der DE-A 198 37 517, DE-A 199 10 506, DE-A 199 10 508 sowie der DE-A 198 37 519 beschrieben ist. Üblicherweise wird die externe Temperierung in den beiden Oxidationszonen, gegebenenfalls in Mehrzonenreaktorsystemen in an sich bekannter Weise an die spezielle Reaktionsgasgemischzusammensetzung sowie Katalysatorbeschickung angepasst.

Erfindungsgemäß vorteilhaft ist, daß bei dem erfindungsgemäßen Verfahren Propenbegleitendes Propan im Rahmen einer heterogen katalysierten Propenoxidation als vorteilhaftes inertes Verdünnungsgas fungiert.

Der für die Oxidation insgesamt benötigte molekulare Sauerstoff kann dem Gas B in seiner Gesamtmenge vorab zugegeben werden. Er kann aber auch nach der ersten Oxidationszone mit Sauerstoff ergänzt werden. Vorzugsweise wird in der ersten Oxidationszone ein molares Verhältnis von Propen : molekularer Sauerstoff von 1 : 1 bis 3, häufig 1 : 1,5 bis 2 eingestellt. In der zweiten Oxidationszone wird vorzugsweise ein molares Verhältnis von Acrolein : molekularer Sauerstoff von 1 : 0,5 bis 2 eingestellt.

In beiden Oxidationszonen wirkt sich ein Überschuss an molekularem Sauerstoff in der Regel vorteilhaft auf die Kinetik der Gasphasenoxidation aus. Da die heterogen katalysierte Gasphasenoxidation des Propens zu Acrylsäure kinetischer Kontrolle unterliegt, kann daher prinzipiell zum Beispiel auch in der ersten Oxidationszone das Propen gegenüber dem molekularem Sauerstoff im molaren Überschuss vorgelegt werden. In diesem Fall kommt dem überschüssigen Propen faktisch die Rolle eines Verdünnungsgases zu.

Prinzipiell ist die heterogen katalysierte Gasphasen-Oxidation von Propen zu Acrylsäure aber auch in einer einzigen Oxidationszone realisierbar. In diesem Fall erfolgen beide Reaktionsschritte in einem Oxidationsreaktor, der mit einem Katalysator beschickt ist, der die Umsetzung beider Reaktionsschritte zu katalysieren vermag. Hierbei kann sich auch die Katalysatorbeschickung innerhalb der Oxidationszone längs der Reaktionskoordinate kontinuierlich oder abrupt ändern. Es kann auch bei einer Ausführungsform des Schrittes (d) in Gestalt zweier hintereinandergeschalteter Oxidationszonen aus dem die erste Oxidationszone verlassenden Produktgasgemisch in selbigem enthaltendes, in der ersten Oxidationszone als Nebenprodukt entstandenes, Kohlenoxid und Wasserdampf bei Bedarf vor der Weiterleitung in die zweite Oxidationszone teilweise oder vollständig abgetrennt werden. Vorzugsweise wird man eine Verfahrensweise wählen, die solch einer Abtrennung nicht bedarf.

Als Quelle für den in dem Oxidationsschritt (d) benötigten molekularen Sauerstoff kommen sowohl reiner molekularer Sauerstoff als auch mit Inertgas, wie Kohlendioxid, Kohlenmonoxid, Edelgasen, Stickstoff und/oder gesättigten Kohlenwasserstoffen verdünnter molekularer Sauerstoff in Betracht.

In zweckmäßiger Weise wird man wenigstens zur Deckung eines Teilbedarfs an molekularem Sauerstoff Luft als Sauerstoffquelle verwenden. Mit Vorteil wird das dem Oxidationsschritt (d) des erfindungsgemäßen Verfahrens zugeführte Gas B im wesentlichen nur aus Propan und Propen bestehen und als Quelle für molekularen Sauerstoff für die Oxidation wird ausschließlich Luft verwendet. Falls erforderlich, kann durch Zudosieren von kalter Luft auch auf direktem Weg eine Abkühlung des Schritt (d) zugeführten Gases B bewirkt werden.

Ist Acrolein das Zielprodukt, wird man in Schritt (d) die zweite Oxidationszone in zweckmäßiger Weise nicht mehr anwenden.

Die Oxidation von Propen zu Acrolein und/oder Acrylsäure in Schritt (d) kann auch durchgeführt werden wie beschrieben in der EP-A-0 117 146, US-A-5 198 578, US-A-5 183 936 oder in Analogie zur DE-A 33 13 573, CA-A-1 217 502, US-A-3 161 670, US-A-4 532 365 und WO 97/36849. Geeignete Verfahren sind auch beschrieben in der EP-A-0 293 224, EP-A-0 253 409, DE-A 44 31 957, DE 195 08 532 oder DE-A 41 32 263, wobei insbesondere die Verfahren bevorzugt sind, die mit Verdünnungsgasen in der Oxidation arbeiten.

Die Oxidation von Acrolein zu Acrylsäure kann wie in der WO 00/39065 beschrieben mittels eines Wirbelschichtreaktors durchgeführt werden.

Die Oxidation von Propen zu Acrolein und/oder Acrylsäure kann auch mit den in der DE-A 199 52 964 beschriebenen Plattenwärmetauscherreaktoren durchgeführt werden.

Es ist prinzipiell auch möglich Propan zu Acrolein und/oder Acrylsäure zu oxidieren. Bei dieser Oxidation wird Propan über einem geeigneten Katalysator in einer oder in mehreren Stufen zu Acrolein und/oder Acrylsäure umgesetzt. Hierfür eignen sich alle dem Fachmann bekannten Verfahren. Ein geeignetes Verfahren ist beispielsweise beschrieben in der JP-A-10 36 311.

Für die heterogen katalysierte Gasphasenoxidation von Propan zu Acrolein und/oder Acrylsäure geeignete Katalysatoren sind Multimetalloxidmassen der allgemeinen Formel (I)

$$MoV_b M^1_c M^2_d O_n \qquad (I),$$

mit

M$^1$ = Te und/oder Sb,
M$^2$ = wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, B, Ce, Sn, Zn, Si und In,
b = 0,01 bis 1,
c = > 0 bis 1, vorzugsweise 0,01 bis 1 und
d = > 0 bis 1, vorzugsweise 0,01 bis 1, und
n = eine Zahl, die durch die Wertigkeit und Häufigkeit der von Sauerstoff verschiedenen Elemente in (I) bestimmt wird.

Multimetalloxidmassen, die eine der allgemeinen Formel (I) entsprechende Stöchiometrie aufweisen, sind bekannt, vergleiche zum Beispiel, EP-A-0 608 838, EP-A-0 529 853, JP-A 7-232 071, JP-A 10-57813, JP-A 2000-37623, JP-A 10-36311, WO 00/29105, Proceedings ISO'99, Rimini (Italy), Sept. 10-11, 1999, G. Centi and S. Perathoner Ed., SCI Pub. 1999, EP-A-0 767 164, Catalysis Today 49 (1999), S.141-153, EP-A-0 962 253, Applied Catalysis A: General 194 bis 195 (2000), S. 479 bis 485, JP-A 11/169716, EP-A-0 895 809, DE-A 198 35 247, JP-A 8-57319, JP-A 10-28862, JP-A 11-43314, JP-A 11-57479; WO 00/29106, JP-A 10-330343, JP-A 11-285637, JP-A 10-310539, JP-A 11-42434, JP-A 11-343261, JP-A 11-343262, WO 99/03825, JP-A 7-53448, JP-A 2000-51693 und JP-A 11-263745.

Besonders geeignet sind die nachfolgend beschriebenen Multimetalloxide (I), (II) und (III).

Bei den Multimetalloxidmassen (I) der Formel (I) ist M$^1$ = Te und/oder Sb; M$^2$ = wenigstens eines der Elemente aus der Gruppe umfassend Nb, Ta, W, Ti, Al, Zr, Cr, Mn, Fe, Ru, Co, Rh, Ni, Pd, Pt, Bi, B und Ce; b = 0,01 bis 1; c = 0,01 bis 1; d = 0,01 bis 1; und n = eine Zahl, die durch die Wertigkeit und Häufigkeit der von Sauerstoff verschiedenen Elemente in (I) bestimmt wird.

Vorzugsweise erfolgt die Herstellung einer Multimetalloxidmasse (I) so, dass man ein Gemisch aus Quellen der elementaren Konstituenten der Multimetalloxidmasse (I) einer hydrothermalen Behandlung unterwirft, den sich dabei neu bildenden Feststoff abtrennt und durch thermische Behandlung in ein aktives Oxid überführt. Bevorzugt ist bei der Multmetalloxidmasse (I) M$^1$ = Te, M$^2$ = Nb, b = 0,1-0,6; c = 0,05-0,4; und d = 0,01-0,6. Vorzugsweise erfolgt die thermische Behandlung bei einer Temperatur von 350 bis 700°C, wobei insbesondere die thermische Behandlung zunächst bei einer Temperatur von 150 bis 400°C unter Sauerstoff-haltiger Atmosphäre und anschließend bei einer Temperatur von 350 bis 700°C unter Inertgasatmosphäre erfolgt. Geeignete Stöchiometrien für die Metalloxidmassen (I) sind jene, die in den Schriften EP-A-0 608 838, WO 00/29106, JP-A 11/169716 und EP-A-0 962 253 offenbart sind.

Die hydrothermale Herstellung von Multimetalloxid-Aktivmassenvorläufem ist dem Fachmann vertraut (vergleiche zum Beispiel Applied Catalysis A: 194 bis 195 (2000) 479-485, Kinetics and Catalysis, Vol. 40, No. 3, 1999, pp. 401 bis 404, Chem. Commun., 1999, 517 bis 518, JP-A 6/227819 und JP-A 2000/26123).

Im besonderen wird hier darunter die thermische Behandlung eines, vorzugsweise innigen, Gemisches von Quellen der elementaren Konstituenten der gewünschten Multimetalloxidmasse (I) in einem Überdruckgefäß (Autoklav) im Beisein von überatmosphärischen Druck aufweisendem Wasserdampf, üblicherweise bei Temperaturen im Bereich von >100°C bis 600°C, verstanden. Der Druckbereich erstreckt sich dabei in typischer Weise auf bis zu 500 atm, vorzugsweise auf bis zu 250 atm. Es können auch Temperaturen oberhalb von 600°C und Wasserdampfdrucke oberhalb von 500 atm angewendet werden, was anwendungstechnisch jedoch wenig zweckmäßig ist. Mit besonderem Vorteil erfolgt die hydrothermale Behandlung unter solchen Bedingungen, unter denen Wasserdampf und flüssiges Wasser koexistieren. Dies ist im Temperaturbereich von >100°C bis 374,15°C (kritische Temperatur des Wassers) unter Anwendung der entsprechenden Drücke möglich. Die Mengen an Wasser werden dabei zweckmäßig so bemessen, dass die flüssige Phase die Gesamtmenge der Ausgangsverbindungen in Suspension und/oder Lösung aufzunehmen vermag.

Es ist aber auch eine solche Verfahrensweise möglich, bei der das innige Gemisch der Ausgangsverbindungen die mit dem Wasserdampf im Gleichgewicht befindliche flüssige Wassermenge vollständig absorbiert.

Vorteilhaft erfolgt die hydrothermale Behandlung bei Temperaturen von >100 bis 300°C, vorzugsweise bei Temperaturen von 150 bis 250°C (zum Beispiel 160 bis 180°C). Bezogen auf die Summe aus Wasser und Quellen der elementaren Konstituenten der gewünschten Multimetalloxidmasse (I) beträgt der Gewichtsanteil der letzteren im Autoklaven in der Regel wenigstens 1 Gew.-%. Üblicherweise liegt der vorgenannte Gewichtsanteil nicht oberhalb von 90 Gew.-%. Typisch sind Gewichtsanteile von 3 bis 60, beziehungsweise von 5 bis 30 Gew.-%, häufig 5 bis 15 Gew.-%.

Während der hydrothermalen Behandlung kann sowohl gerührt als auch nicht gerührt werden. Zur hydrothermalen Herstellvariante kommen als Ausgangsverbindungen (Quellen) insbesondere alle diejenigen in Betracht, die beim Erhitzen unter Überdruck mit Wasser Oxide und/oder Hydroxide zu bilden vermögen. Es können als Ausgangsverbindungen für die hydrothermale Herstellung auch bereits Oxide und/oder Hydroxide der elementaren Konstituenten mitverwendet oder ausschließlich verwendet werden. In der Regel werden die Quellen in feinteiliger Form eingesetzt.

Als Quellen für die elementaren Konstituenten kommen alle diejenigen in Betracht, die beim Erhitzen (gegebenenfalls an Luft) Oxide und/oder Hydroxide zu bilden vermögen. Es können als solche Ausgangsverbindungen auch bereits Oxide und/oder Hydroxide der elementaren Konstituenten mitverwendet oder ausschließlich verwendet werden.

Geeignete Quellen für das Element Mo sind zum Beispiel Molybdänoxide wie Molybdäntrioxid, Molybdate wie Ammoniumheptamolybdattetrahydrat und Molybdänhalogenide wie Molybdänchlorid.

Geeignete mit zu verwendende Ausgangsverbindungen für das Element V sind zum Beispiel Vanadylacetylacetonat, Vanadate wie Ammoniummetavandat, Vanadinoxide wie Vanadinpentoxid ($V_2O_5$), Vanadinhalogenide wie Vanadintetrachlorid ($VCl_4$) und Vanadinoxyhalogenide wie $VOCl_3$. Zweckmäßigerweise werden als Vanadinausgangsverbindungen solche mitverwendet, die das Vanadin in der Oxidationsstufe +4 enthalten.

Als Quellen für das Element Tellur eignen sich Telluroxide wie Tellurdioxid, metallisches Tellur, Tellurhalogenide wie $TeCl_2$, aber auch Tellursäuren wie Orthotellursäure $H_6TeO_6$.

Vorteilhafte Antimonausgangsverbindungen sind Antimonhalogenide, wie $SbCl_3$, Antimonoxide wie Antimontrioxid ($Sb_2O_3$), Antimonsäuren wie $HSb(OH)_6$, aber auch Antimonoxid-salze, wie Antimonoxid-sulfat ($SbO_2)SO_4$. Geeignete Niobquellen sind zum Beispiel Nioboxide wie Niobpentoxid ($Nb_2O_5$), Nioboxidhalogenide wie $NbOCl_3$, Niobhalogenide wie $NbCl_5$, aber auch komplexe Verbindungen aus Niob und organischen Carbonsäuren und/oder Dicarbonsäuren wie zum Beispiel Oxalate und Alkoholate. Als Niobquelle kommen auch die in der EP-A-0 895 809 verwendeten Nb enthaltenden Lösungen in Betracht.

Bezüglich aller anderen möglichen Elemente $M^2$ kommen als geeignete Ausgangsverbindungen vor allem deren Halogenide, Nitrate, Formiate, Oxalate, Acetate, Carbonate und/oder Hydroxide in Betracht. Geeignete Ausgangsverbindungen sind vielfach auch deren Oxoverbindungen wie zum Beispiel Wolframate beziehungsweise die von diesen abgeleiteten Säuren. Häufig werden als Ausgangsverbindungen auch Ammoniumsalze eingesetzt.

Ferner kommen als Ausgangsverbindungen auch Polyanionen vom Anderson-Typ in Betracht, wie sie zum Beispiel in Polyhedron Vol. 6, No. 2, pp. 213-218, 1987 beschrieben und zum Beispiel in Applied Catalysis A: General 194-195 (2000) 479-485 zur Herstellung von geeigneten Multimetalloxiden (I) verwendet worden beziehungsweise in der darin zitierten Sekundärliteratur offenbart sind. Eine weitere geeignete Literaturquelle für Polyanionen vom Anderson-Typ bildet Kinetics and Catalysis, Vol. 40, No. 1999, pp. 401 bis 404.

Weitere als Ausgangsverbindungen geeignete Polyanionen sind zum Beispiel solche vom Dawson- oder Keggin-Typ. Vorzugsweise werden solche Ausgangsverbindungen verwendet, die sich bei erhöhten Temperaturen entweder im Beisein oder bei Ausschluß von Sauerstoff, gegebenenfalls unter Freisetzung gasförmiger Verbindungen, in ihre Oxide umwandeln.

Die hydrothermale Behandlung selbst nimmt in der Regel eine Zeitdauer von wenigen Stunden bis zu einigen Tagen in Anspruch. Typisch ist ein Zeitraum von 48 h. Anwendungstechnisch zweckmäßig ist der für die hydrothermale Behandlung zu verwendende Autoklav innenseitig mit Teflon beschichtet. Vorab der hydrothermalen Behandlung kann der Autoklav, gegebenenfalls einschließlich des enthaltenen wäßrigen Gemisches, evakuiert werden. Anschließend kann vor der Temperaturerhöhung mit Inertgas ($N_2$; Edelgas) gefüllt werden. Beide Maßnahmen können auch unterlassen werden. Das wäßrige Gemisch kann zur Inertisierung vorab einer hydrothermalen Behandlung zusätzlich mit Inertgas gespült werden. Die vorgenannten Inertgase können anwendungstechnisch zweckmäßig auch dazu benutzt werden, bereits vorab der hydrothermalen Behandlung im Autoklaven überatmosphärischen Druck einzustellen. Die erforderliche Behandlung des sich im Verlauf der hydrothermalen Behandlung neu gebildeten und nach Beendigung der hydrothermalen Behandlung (nach Beendigung der hydrothermalen Behandlung kann der Autoklav entweder auf Raumtemperatur abgeschreckt oder langsam, das heißt über einen längeren Zeitraum (zum Beispiel durch sich selbst überlassen) auf Raumtemperatur gebracht werden) abgetrennten Feststoffs wird zweckmäßigerweise bei einer Temperatur von 350 bis 700°C, häufig bei einer Temperatur von 400 bis 650°C, beziehungsweise 400 bis 600°C durchgeführt. Sie kann sowohl unter oxidierender, reduzierender als auch unter inerter Atmosphäre erfolgen. Als oxidierende Atmosphäre kommt zum Beispiel Luft, mit molekularem Sauerstoff angereicherte Luft oder an Sauerstoff entreicherte Luft in Betracht. Vorzugsweise wird die thermische Behandlung unter inerter Atmosphäre, das heißt, zum Beispiel unter molekularem Stickstoff und/oder Edelgas, durchgeführt. Selbstverständlich kann die thermische Behandlung auch unter Vakuum erfolgen.

Erfolgt die thermische Behandlung unter gasförmiger Atmosphäre, kann diese sowohl stehen als auch fließen.

Insgesamt kann die thermische Behandlung bis zu 24 h oder mehr in Anspruch nehmen.

Bevorzugt erfolgt die thermische Behandlung zunächst unter oxidierender (Sauerstoff enthaltender) Atmosphäre (zum Beispiel unter Luft) bei einer Temperatur von 150 bis 400°C beziehungsweise 250 bis 350°C. Im Anschluß daran wird die thermische Behandlung zweckmäßig unter Inertgas bei Temperaturen von 350 bis 700°C beziehungsweise 400 bis 650°C oder 400 bis 600°C fortgesetzt. Die thermische Behandlung des hydrothermal erzeugten Katalysatorvorläufers kann auch so erfolgen, dass die Katalysatorvorläufermasse zunächst tablettiert, dann thermisch behandelt und nachfolgend versplittet wird.

Anwendungstechnisch zweckmäßig wird der im Rahmen der hydrothermalen Behandlung gewonnene Feststoff zur nachfolgenden thermischen Behandlung versplittet.

Verwendet man für die Herstellung der Multimetalloxidmassen (I) die gleichen Ausgangsverbindungen wie für eine konventionelle Herstellung von Multimetalloxiden (I) und führt man die thermische Behandlung des in konventioneller Weise erzeugten innigen Trocknungsgemisches in gleicher Weise durch, wie die thermische Behandlung des hydrothermal gewonnenen Feststoffs, weisen die Multimetalloxidmassen (I) bezüglich der heterogen katalysierten Gasphasenoxidation von Propan zu Acrylsäure unter gleichen Bedingungen in der Regel eine höhere Selektivität der Acrylsäurebildung sowie eine erhöhte Aktivität auf.

Die Multimetalloxidmassen (I) können als solche (zum Beispiel zu Pulver oder zu Splitt zerkleinert) oder auch zu Förmkörpern geformt eingesetzt werden. Dabei kann das Katalysatorbett sowohl ein Festbett, ein Wanderbett oder ein Wirbelbett sein.

Das Röntgendiffraktogramm der Multimetalloxidmassen (I) entspricht in der Regel im wesentlichen jenen aus den Schriften EP-A-0 529 853, DE-A 198 35 247 und EP-A-0 608 838.

Die Multimetalloxidmassen (I) können auch in mit feinteiligen, zum Beispiel kolloidalen, Materialien wie Siliciumdioxid, Titandioxid, Aluminiumoxid, Zirkonoxid, Nioboxid verdünnter Form eingesetzt werden.

Das Verdünnungsmassenverhältnis kann dabei bis zu 9 (Verdünner) : 1 (Aktivmasse) betragen. Das heißt, mögliche Verdünnungsmassenverhältnisse sind auch 6 (Verdünner) : 1 (Aktivmasse) und 3 (Verdünner) : 1 (Aktivmasse). Die Einarbeitung der Verdünner kann vor und/oder nach der Calcination erfolgen. In der Regel erfolgt die Einarbeitung des Verdünners vor der hydrothermalen Behandlung. Erfolgt die Einarbeitung vor der Calcination, muss der Verdünner so gewählt werden, dass er bei der Calcination als solcher im wesentlichen erhalten bleibt. Das gleiche gilt bezüglich der hydrothermalen Behandlung bei einer Einarbeitung vor Durchführung derselben. Dies ist zum Beispiel im Fall von bei entsprechend hohen Temperaturen gebrannten Oxiden in der Regel gegeben.

Weiterhin für die Propanoxidation geeignete Katalysatoren sind Multimetalloxidmassen (II), die die oben angegebene Formel (I) aufweisen und bei denen das Röntgendiffraktogramm Beugungsreflexe h, i und k aufweist, deren Scheitelpunkte bei den Beugungswinkeln (2θ) 22,2 ± 0,4° (h), 27,3 ± 0,4° (i) und 28,2 ± 0,4° (k) liegen, wobei

- der Beugungsreflex h innerhalb des Röntgendiffraktogramms der intensitätsstärkste ist sowie eine Halbwertsbreite von höchstens 0,5° aufweist,
- die Intensität $P_i$ des Beugungsreflexes i und die Intensität $P_k$ des Beugungsreflexes k die Beziehung $0,65 \leq R \leq 0,85$ erfüllen, in der R das durch die Formel

$$R = P_i / (P_i + P_k)$$

definierte Intensitätsverhältnis ist, und
- die Halbwertsbreite des Beugungsreflexes i und des Beugungsreflexes k jeweils ≤1° beträgt.

Bevorzugt gilt $0,67 \leq R \leq 0,75$ und ganz besonders bevorzugt gilt R = 0,70 bis 0,75 beziehungsweise R = 0,72.

Bevorzugt ist die Verwendung von Multimetalloxidmassen (II) mit $M^1$ = Te. Ferner sind solche Multimetalloxidmassen (II) günstig, bei denen $M^2$ = Nb, Ta, W und/oder Titan ist. Vorzugsweise ist $M^2$ = Nb.

Der stöchiometrische Koeffizient b der Multimetalloxidmassen (II) beträgt mit Vorteil 0,1 bis 0,6. In entsprechender Weise beläuft sich der Vorzugsbereich für den stöchiometrischen Koeffizienten c auf 0,01 bis 1 beziehungsweise auf 0,05 bis 0,4 und günstige Werte für d betragen 0,01 bis 1 beziehungsweise 0,1 bis 0,6. Besonders günstige Multimetalloxidmassen (II) sind solche, bei denen die stöchiometrischen Koeffizienten b, c und d simultan in den vorgenannten Vorzugsbereichen liegen. Weitere geeignete Stöchiometrien für die Multimetalloxidmassen (II) sind jene, die in den Schriften des zuvor zitierten Standes der Technik, insbesondere solche, die der JP-A 7-53448 offenbart sind.

Ein gezieltes Verfahren zur Herstellung von Multimetalloxidmassen (II) offenbart zum Beispiel die JP-A 11-43314, in welcher die relevanten Multimetalloxidmassen (II) als Katalysatoren für die heterogen katalysierte Oxidehydrierung von Ethan zu Ethen empfohlen werden.

Danach wird in an sich bekannter, in den zitierten Schriften des Standes der Technik offenbarter, Weise zunächst eine Multimetalloxidmasse der Formel (I) erzeugt, die ein Gemisch aus i-Phase und anderen Phasen (zum Beispiel k-Phase) ist. In diesem Gemisch kann nun zum Beispiel der Anteil an i-Phase dadurch erhöht werden, dass man die anderen Phasen, zum Beispiel die k-Phase, unter dem Mikroskop ausliest, oder die Multimetalloxidmasse mit geeigneten Flüssigkeiten wäscht. Als solche Flüssigkeiten kommen zum Beispiel wäßrige Lösungen organischer Säuren (zum Beispiel Oxalsäure, Ameisensäure, Essigsäure, Zitronensäure und Weinsäure), anorganischer Säuren (zum Beispiel Salpetersäure), Alkohole und wäßrige Wasserstoffperoxidlösungen in Betracht. Des weiteren offenbart auch die JP-A 7-232071 ein Verfahren zur Herstellung von Multimetalloxidmassen (II).

Multimetalloxidmassen (II) sind durch die Herstellweise gemäß DE-A 198 35 247 erhältlich. Danach wird von geeigneten Quellen ihrer elementaren Konstituenten ein möglichst inniges, vorzugsweise feinteiliges, Trockengemisch erzeugt und dieses bei Temperaturen von 350 bis 700°C beziehungsweise 400 bis 650°C oder 400 bis 600°C thermisch behandelt. Die thermische Behandlung kann sowohl unter oxidierender, reduzierender als auch unter inerter Atmosphäre erfolgen. Als oxidierende Atmosphäre kommt zum Beispiel Luft, mit molekularem Sauerstoff angereicherte Luft oder an Sauerstoff entreicherte Luft in Betracht. Vorzugsweise wird die thermische Behandlung unter inerter Atmosphäre, das heißt zum Beispiel unter molekularem Stickstoff und/oder Edelgas, durchgeführt. Üblicherweise erfolgt die thermische Behandlung bei Normaldruck (1 atm). Selbstverständlich kann die thermische Behandlung auch unter Vakuum oder unter Überdruck erfolgen.

Erfolgt die thermische Behandlung unter gasförmiger Atmosphäre, kann diese sowohl stehen als auch fließen. Insgesamt kann die thermische Behandlung bis zu 24 h oder mehr in Anspruch nehmen.

Bevorzugt erfolgt die thermische Behandlung zunächst unter oxidierender (Sauerstoff enthaltender) Atmosphäre (zum Beispiel unter Luft) bei einer Temperatur von 150 bis 400°C beziehungsweise 250 bis 350°C. Im Anschluß daran wird die thermische Behandlung zweckmäßig unter Inertgas bei Temperaturen von 350 bis 700°C beziehungsweise 400 bis 650°C oder 400 bis 600°C fortgesetzt. Die thermische Behandlung kann auch so erfolgen, dass die Katalysatorvorläufermasse vor ihrer thermischen Behandlung zunächst (gegebenenfalls nach Pulverisierung) tablettiert (gegebenenfalls unter Zusatz von 0,5 bis 2 Gew.-% an feinteiligem Graphit), dann thermisch behandelt und nachfolgend wieder versplittet wird.

Das innige Vermischen der Ausgangsverbindungen im Rahmen der Herstellung der Multimetalloxidmassen (II) kann in trockener oder in nasser Form erfolgen. Erfolgt es in trockener Form, werden die Ausgangsverbindungen zweckmäßigerweise als feinteilige Pulver eingesetzt und nach dem Mischen und gegebenenfalls Verdichten der Calcinierung (thermischen Behandlung) unterworfen. Vorzugsweise erfolgt das innige Vermischen jedoch in nasser Form. Üblicherweise werden die Ausgangsverbindungen dabei in Form einer wäßrigen Lösung und/oder Suspension miteinander vermischt. Anschließend wird die wäßrige Masse getrocknet und nach der Trocknung calciniert. Zweckmäßigerweise handelt es sich bei der wäßrigen Masse um eine wäßrige Lösung oder um eine wäßrige Suspension. Vorzugsweise erfolgt der Trocknungsprozess unmittelbar im Anschluß an die Herstellung der wäßrigen Mischung und durch Sprühtrocknung (die Austrittstemperaturen betragen in der Regel 100 bis 150°C; die Sprühtrocknung kann im Gleichstrom oder im Gegenstrom durchgeführt werden), die ein besonders inniges Trockengemisch bedingt, vor allem dann, wenn es sich bei der sprühzutrocknenden wäßrigen Masse um eine wäßrige Lösung oder Suspension handelt.

Als Quellen oder Ausgangsverbindungen für die Multimetalloxidmasse (II) eignen sich die oben bei der Multimetalloxidmasse (I) beschriebenen Verbindungen.

Die Multimetalloxidmassen (II) können ebenso wie die Multimetalloxidmassen (I) zu Formkörpern geformt und wie diese eingesetzt werden. Die Formgebung der Multimetalloxidmassen (II) kann beispielsweise durch Aufbringen auf einen Träger, wie nachstehend unter Katalysator (III) beschrieben, oder durch Extrusion und/oder Tablettierung sowohl von feinteiliger Multimetalloxidmasse (II) als auch von feinteiliger Vorläufermasse einer Multimetalloxidmasse (II) erfolgen.

In gleicher Weise wie die Multimetalloxidmassen (I) können die Multimetalloxidmassen (II) auch in mit feinteiligen Materialien verdünnter Form eingesetzt werden.

Als Geometrien kommen dabei sowohl Kugeln, Vollzylinder und Hohlzylinder (Ringe) in Betracht. Die Längstausdehnung der vorgenannten Geometrien beträgt dabei in der Regel 1 bis 10 mm. In Fall von Zylindern beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Geeignete ringförmige Vollkatalysatoren können auch eine Länge von 3 bis 6 mm, einen Außendurchmesser von 4 bis 8 mm und eine Wanddicke von 1 bis 2 mm aufweisen. Möglich ist aber auch eine Vollkatalysatorringgeometrie von 7 mm x 3 mm x 4 mm oder von 5 mm x 3 mm x 2 mm (Außendurchmesser x Länge x Innendurchmesser).

Die Definition der Intensität eines Beugungsreflexes im Röntgendiffraktogramm bezieht sich vorliegend auf die in der DE-A 198 35 247, sowie die in der DE-A 100 51 419 und DE-A 100 46 672 niedergelegte Definition.

Das heißt, bezeichnet $A^1$ den Scheitelpunkt eines Reflexes 1 und bezeichnet $B^1$ in der Linie des Röntgendiffraktogramms bei Betrachtung entlang der zu 2θ-Achse senkrecht stehenden Intensitätsachse das nächstliegende ausgeprägte Minimum (Reflexschultern ausweisende Minima bleiben unberücksichtigt) links vom Scheitelpunkt $A^1$ und $B^2$ in entsprechender Weise das nächstliegende ausgeprägte Minimum rechts vom Scheitelpunkt $A^1$ und bezeichnet $C^1$ einen Punkt, an dem eine vom Scheitelpunkt $A^1$ senkrecht zur 26-Achse gezogene Gerade eine die Punkte $B^1$ und $B^2$

verbindende Gerade schneidet, dann ist die Intensität des Reflexes 1 die Länge des Geradenabschnitts $A^1C^1$, der sich vom Scheitelpunkt $A^1$ zum Punkt $C^1$ erstreckt. Der Ausdruck Minimum bedeutet dabei einen Punkt, an dem der Steigungsgradient einer an die Kurve in einem Basisbereich des Reflexes 1 angelegten Tangente von einem negativen Wert auf einen positiven Wert übergeht, oder einen Punkt, an dem der Steigungsgradient gegen Null geht, wobei für die Festlegung des Steigungsgradienten die Koordinaten der 2θ-Achse und der Intensitätsachse herangezogen werden.

Die Halbwertsbreite ist vorliegend in entsprechender Weise die Länge des Geradenabschnitts, der sich zwischen den beiden Schnittpunkten $H^1$ und $H^2$ ergibt, wenn man in der Mitte des Geradenabschnitts $A^1C^1$ eine Parallele zur 2θ-Achse zieht, wobei $H^1$, $H^2$ den jeweils ersten Schnittpunkt dieser Parallelen mit der wie vorstehend definierten Linie des Röntgendiffraktogramms-links und rechts von $A^1$ meinen.

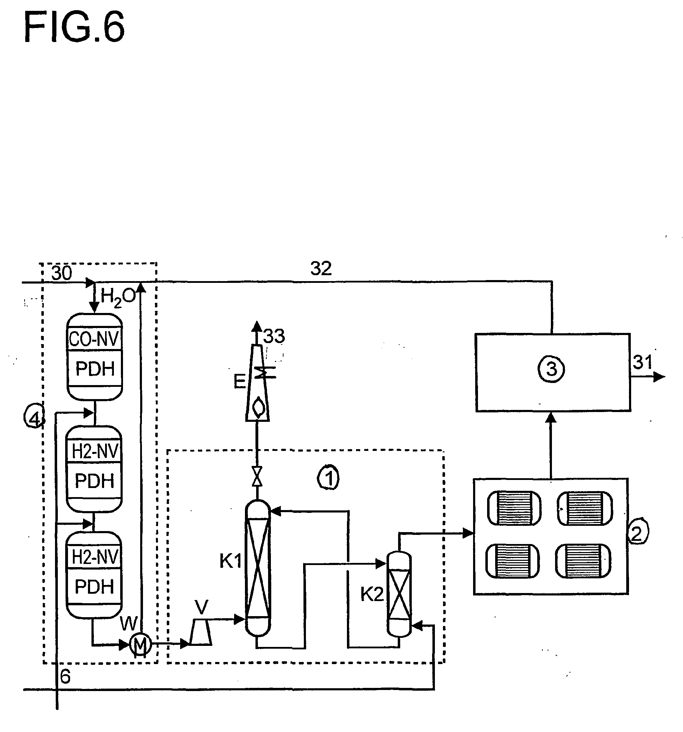

Eine beispielhafte Durchführung der Bestimmung von Halbwertsbreite und Intensität zeigt auch die Figur 6 in der DE-A 100 46 672.

Neben den Beugungsreflexen h, i und k enthält das Röntgendiffraktogramm vorteilhafter katalytisch aktiver Multimetalloxidmassen (II) in der Regel noch weitere Beugungsreflexe, deren Scheitelpunkte bei den nachfolgenden Beugungswinkeln (2θ) liegen:

$$9,0 \pm 0,4° \text{ (l),}$$

$$6,7 \pm 0,4° \text{ (o)}$$

und

$$7,9 \pm 0,4° \text{ (p).}$$

Günstig ist es, wenn das Röntgendiffraktogramm der katalytisch aktiven Oxidmassen der allgemeinen Formel (I) zusätzlich einen Beugungsreflex enthält, dessen Scheitelpunkt bei folgendem Beugungswinkel (2θ) liegt:

$$45,2 \pm 0,4° \text{ (q).}$$

Häufig enthält das Röntgendiffraktogramm der Multimetalloxidmassen (II) auch noch die Reflexe $29,2 \pm 0,4°$ (m) und $35,4 \pm 0,4°$ (n).

Die Multimetalloxidmasse (II) kann eine solche sein, deren Röntgendiffraktogramm keinen Beugungsreflex mit der Scheitelpunktlage $2\theta = 50,0 \pm 0,3°$ aufweist, das heißt, eine solche sein, die keine k-Phase enthält.

Die Multimetalloxidmasse (II) kann jedoch auch k-Phase enthalten, wobei ihr Röntgendiffraktogramm in der Regel noch weiter Beugungsreflexe enthält, deren Scheitelpunkte bei den nachfolgenden Beugungswinkeln (2θ) liegen:

$$36,2 \pm 0,4°$$

und

$$50,0 \pm 0,4°.$$

Ordnet man dem Beugungsreflex h die Intensität 100 zu, ist es günstig; wenn die Beugungsreflexe i, l, m, n, o, p, q in der gleichen Intensitätsskala die nachfolgenden Intensitäten aufweisen:

i: 5 bis 95, häufig 5 bis 80, teilweise 10 bis 60;
l: 1 bis 30;

m: 1 bis 40;

n: 1 bis 40;

o: 1 bis 30;

p: 1 bis 30 und

q: 5 bis 60.

Enthält das Röntgendiffraktogramm von den vorgenannten zusätzlichen Beugungsreflexen, ist die Halbwertsbreite derselben in der Regel ≤ 1°.

Alle vorliegend auf ein Röntgendiffraktogramm bezogenen Angaben beziehen sich auf ein unter Anwendung von Cu-K$\alpha$-Strahlung als Röntgenstrahlung erzeugtes Röntgendiffraktogramm (Siemens-Diffraktometer Theta-Theta D-5000, Röhrenspannung: 40 kV, Röhrenstrom: 40 mA, Aperturblende V20 (variabel), Streustrahlblende V20 (variabel), Sekundärmonochromatorblende (0,1 mm), Detektorblende (0,6 mm), Meßintervall (2θ): 0,02°, Meßzeit je Schritt: 2,4 s, Detektor: Szintillationszählrohr).

Die spezifische Oberfläche von Multimetalloxidmassen (II) beträgt vielfach 1 bis 30 m$^2$/g (BET-Oberfläche, Stickstoff).

Als Katalysator für die Propanoxidation eignet sich auch ein Katalysator (III), der aus einem Trägerkörper und einer auf der Oberfläche des Trägerkörpers aufgebrachten katalytisch aktiven Oxidmasse der oben genannten Formel (I) besteht.

Bevorzugt ist die Verwendung von Oxidmassen der allgemeinen Formel (I) mit M$^1$ = Te. Ferner ist es günstig, wenn M$^2$ = Nb, Ta, W und/oder Titan ist. Vorzugsweise ist M$^2$ = Nb.

Der stöchiometrische Koeffizient b der Oxidmassen der allgemeinen Formel (I) beträgt in Katalysator (III) mit Vorteil 0,1 bis 0,6. In entsprechender Weise beläuft sich der Vorzugsbereich für den stöchiometrischen Koeffizienten c auf 0,01 bis 1 beziehungsweise auf 0,05 bis 0,4 und günstige Werte für d betragen 0,01 bis 1 beziehungsweise 0,1 bis 0,6. Besonders günstige Oxidmassen sind solche, bei denen die stöchiometrischen Koeffizienten b, c und d simultan in den vorgenannten Vorzugsbereichen liegen.

Weitere geeignete Stöchiometrien für die Oxidmassen der allgemeinen Formel (I) sind jene, die in den obengenannten Schriften, insbesondere solche, die in EP-A-0 608 838, WO 00/29106, JP-A 11/169716 und EP-A-0 962 253, offenbart sind.

Besonders bevorzugt ist zur Herstellung des Katalysators (III) auch das Aufbringen der oben beschriebenen Multimetalloxidmasse (II) als der Oxidmasse der Formel (I) auf einen Trägerkörper.

Die Trägerkörper sind vorzugsweise chemisch inert, das heißt sie greifen in den Ablauf der katalytischen Gasphasenoxidation des Propans zu Acrylsäure im wesentlichen nicht ein. Als Material für die Trägerkörper kommen insbesondere Aluminiumoxid, Siliciumdioxid, Silicate wie Ton, Kaolin, Steatit, Bims, Aluminiumsilicat und Magnesiumsilicat, Siliciumcarbid, Zirkondioxid und Thoriumdioxid in Betracht.

Die Oberfläche des Trägerkörpers kann sowohl glatt als auch rauh sein. Mit Vorteil ist die Oberfläche des Trägerkörpers rauh, da eine erhöhte Oberflächenrauhigkeit in der Regel eine erhöhte Haftfestigkeit der aufgebrachten Aktivmassenschale bedingt.

Häufig liegt die Oberflächenrauhigkeit R$_z$ des Trägerkörpers im Bereich von 5 bis 200 $\mu$m, oft im Bereich von 20 bis 100 $\mu$m (bestimmt gemäß DIN 4768 Blatt 1 mit einem "Hommel Tester für DIN-ISO Oberflächenmeßgrößen" der Firma Hommelwerke, DE).

Ferner kann das Trägermaterial porös oder unporös sein. Zweckmäßigerweise ist das Trägermaterial unporös (Gesamtvolumen der Poren auf das Volumen des Trägerkörpers bezogen ≤ 1 Vol.-%).

Die Dicke der auf dem Schalenkatalysator befindlichen aktiven Oxidmassenschale liegt üblicherweise bei 10 bis 1000 mm. Sie kann aber auch 50 bis 700 $\mu$m, 100 bis 600 $\mu$m oder 300 bis 500 $\mu$m oder 150 bis 400 $\mu$m betragen. Mögliche Schalendicken sind auch 10 bis 500 $\mu$m, 100 bis 500 $\mu$m oder 150 bis 300 $\mu$m.

Prinzipiell kommen beliebige Geometrien der Trägerkörper in Betracht. Ihre Längsausdehnung beträgt in der Regel 1 bis 10 mm. Vorzugsweise werden jedoch Kugeln oder Zylinder, insbesondere Hohlzylinder (Ringe), als Trägerkörper angewendet. Günstige Durchmesser für Trägerkugeln betragen 1,5 bis 4 mm. Werden Zylinder als Trägerkörper verwendet, so beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Geeignete ringförmige Trägerkörper können auch eine Länge von 3 bis 6 mm, einen Außendurchmesser von 4 bis 8 mm und eine Wanddicke von 1 bis 2 mm aufweisen. Möglich ist aber auch eine Trägerringgeometrie von 7 mm x 3 mm x 4 mm oder von 5 mm x 3 mm x 2 mm (Außendurchmesser x Länge x Innendurchmesser).

Die Herstellung der Katalysatoren (III) kann in einfachster Weise so erfolgen, dass man aktive Oxidmassen der allgemeinen Formel (I) vorbildet, sie in eine feinteilige Form überführt und abschließend mit Hilfe eines flüssigen Bindemittels auf die Oberfläche des Trägerkörpers aufbringt. Dazu wird die Oberfläche des Trägerkörpers in einfachster Weise mit dem flüssigen Bindemittel befeuchtet und durch Inkontaktbringen mit feinteiliger aktiver Oxidmasse der allgemeinen Formel (I) eine Schicht der Aktivmasse auf der befeuchteten Oberfläche angeheftet. Abschließend wird der beschichtete Trägerkörper getrocknet. Selbstverständlich kann man zur Erzielung einer erhöhten Schichtdicke den Vorgang periodisch wiederholen. In diesem Fall wird der beschichtete Grundkörper zum neuen "Trägerkörper" etc..

Die Feinheit der auf die Oberfläche des Trägerkörpers aufzubringenden katalytisch aktiven Oxidmasse der allgemeinen Formel (I) wird an die gewünschte Schalendicke angepaßt. Für den Schalendickenbereich von 100 bis 500 $\mu$m eignen sich zum Beispiel solche Aktivmassenpulver, von denen wenigstens 50% der Gesamtzahl der Pulverpartikel ein Sieb der Maschenweite 1 bis 20 $\mu$m passieren und deren numerischer Anteil an Partikeln mit einer Längstausdehnung oberhalb von 50 $\mu$m weniger als 10% beträgt. In der Regel entspricht die Verteilung der Längstausdehnungen der Pulverpartikel herstellungsbedingt einer Gaußverteilung.

Für eine Durchführung des beschriebenen Beschichtungsverfahrens im technischen Maßstab empfiehlt sich zum Beispiel die Anwendung des in der DE-A 29 096 71 offenbarten Verfahrensprinzips. Das heißt, die zu beschichtenden Trägerkörper werden in einem vorzugsweise geneigten (der Neigungswinkel beträgt in der Regel $\geq 0°$ und $\leq 90°$, meist $\geq 30°$ und $\leq 90°$; der Neigungswinkel ist der Winkel der Drehbehälterrnittelachse gegen die Horizontale) rotierenden Drehbehälter (zum Beispiel Drehteller oder Dragiertrommel) vorgelegt. Der rotierende Drehbehälter führt die zum Beispiel kugelförmigen oder zylindrischen Trägerkörper unter zwei in bestimmtem Abstand aufeinanderfolgend angeordneten Dosiervorrichtungen hindurch. Die erste der beiden Dosiervorrichtungen entspricht zweckmäßig einer Düse (zum Beispiel eine mit Druckluft betriebene Zerstäuberdüse), durch die die im rotierenden Drehteller rollenden Trägerkörper mit dem flüssigen Bindemittel besprüht und kontrolliert befeuchtet werden. Die zweite Dosiervorrichtung befindet sich außerhalb des Zerstäubungskegels des eingesprühten flüssigen Bindemittels und dient dazu, die feinteilige oxidische Aktivmasse zuzuführen (zum Beispiel über eine Schüttelrinne oder eine Pulverschnecke). Die kontrolliert befeuchteten Trägerkugeln nehmen das zugeführte Aktivmassenpulver auf, das sich durch die rollende Bewegung auf der äußeren Oberfläche des zum Beispiel zylinder- oder kugelförmigen Trägerkörpers zu einer zusammenhängenden Schale verdichtet.

Bei Bedarf durchläuft der so grundbeschichtete Trägerkörper im Verlauf der darauffolgenden Umdrehung wiederum die Sprühdüsen, wird dabei kontrolliert befeuchtet, um im Verlauf der Weiterbewegung eine weitere Schicht feinteiliger oxidischer Aktivmasse aufnehmen zu können und so weiter (eine Zwischentrocknung ist in der Regel nicht erforderlich). Feinteilige oxidische Aktivmasse und flüssiges Bindemittel werden dabei in der Regel kontinuierlich und simultan zugeführt.

Die Entfernung des flüssigen Bindemittels kann nach beendeter Beschichtung zum Beispiel durch Einwirkung von heißen Gasen, wie $N_2$ oder Luft, erfolgen. Bemerkenswerterweise bewirkt das beschriebene Beschichtungsverfahren sowohl eine voll befriedigende Haftung der aufeinanderfolgenden Schichten aneinander, als auch der Grundschicht auf der Oberfläche des Trägerkörpers.

Wesentlich für die vorstehend beschriebene Beschichtungsweise ist, dass die Befeuchtung der zu beschichtenden Oberfläche des Trägerkörpers in kontrollierter Weise vorgenommen wird. Kurz ausgedrückt heißt dies, dass man die Trägeroberfläche zweckmäßig so befeuchtet, dass diese zwar flüssiges Bindemittel adsorbiert aufweist, aber auf der Trägeroberfläche keine Flüssigphase als solche visuell in Erscheinung tritt. Ist die Trägerkörperoberfläche zu feucht, agglomeriert die feinteilige katalytisch aktive Oxidmasse zu getrennten Agglomeraten, anstatt auf die Oberfläche aufzuziehen. Detaillierte Angaben hierzu finden sich in der DE-A 29 09 671.

Die vorerwähnte abschließende Entfernung des verwendeten flüssigen Bindemittels kann in kontrollierter Weise zum Beispiel durch Verdampfen und/oder Sublimieren vorgenommen werden. Im einfachsten Fall kann dies durch Einwirkung heißer Gase entsprechender Temperatur (häufig 50 bis 300, häufig 150°C) erfolgen. Durch Einwirkung heißer Gase kann aber auch nur eine Vortrocknung bewirkt werden. Die Endtrocknung kann dann beispielsweise in einem Trockenofen beliebiger Art (zum Beispiel Bandtrockner) oder im Reaktor erfolgen. Die einwirkende Temperatur sollte dabei nicht oberhalb der zur Herstellung der oxidischen Aktivmasse angewendeten Calcinationstemperatur liegen. Selbstverständlich kann die Trocknung auch ausschließlich in einem Trockenofen durchgeführt werden.

Als Bindemittel für den Beschichtungsprozess können unabhängig von der Art und der Geometrie des Trägerkörpers verwendet werden: Wasser, einwertige Alkohole wie Ethanol, Methanol, Propanol und Butanol, mehrwertige Alkohole wie Ethylenglykol, 1,4-Butandiol, 1,6-Hexandiol oder Glycerin, ein- oder mehrwertige organische Carbonsäuren wie Propionsäure, Oxalsäure, Malonsäure, Glutarsäure oder Maleinsäure, Aminoalkohole wie Ethanolamin oder Diethanolamin sowie ein- oder mehrwertige organische Amide wie Formamid. Günstige Bindemittel sind auch Lösungen, bestehend aus 20 bis 90 Gew.-% Wasser und 10 bis 80 Gew.-% einer in Wasser gelösten organischen Verbindung, deren Siedepunkt oder Sublimationstemperatur bei Normaldruck (1 atm) > 100°C, vorzugsweise > 150°C, beträgt. Mit Vorteil wird die organische Verbindung aus der vorstehenden Auflistung möglicher organischer Bindemittel ausgewählt. Vorzugsweise beträgt der organische Anteil an vorgenannten wäßrigen Bindemittellösungen 10 bis 50 und besonders bevorzugt 20 bis 30 Gew.-%. Als organische Komponenten kommen dabei auch Monosaccharide und Oligosaccharide wie Glucose, Fructose, Saccharose oder Lactose sowie Polyethylenoxide und Polyacrylate in Betracht.

Die Herstellung von katalytisch aktiven Oxidmassen der allgemeinen Formel (I) kann in an sich bekannter Weise wie in den oben zitierten Schriften des Standes der Technik erfolgen. Das heißt, die Herstellung kann zum Beispiel sowohl hydrothermal als auch auf konventionelle Art und Weise erfolgen.

Im letzteren Fall sind die katalytisch aktiven Oxidmassen der allgemeinen Formel (I) dadurch erhältlich, dass man von geeigneten Quellen ihrer elementaren Konstituenten ein möglichst inniges, vorzugsweise feinteiliges Trockengemisch erzeugt und dieses bei Temperaturen von 350 bis 700°C beziehungsweise 400 bis 650°C oder 400 bis 600°C thermisch behandelt. Die thermische Behandlung kann wie oben bei der Multimetalloxidmasse (II) beschrieben erfolgen. Ebenso kann das innige Vermischen der Ausgangsverbindungen wie oben bei Multimetalloxidmasse (II) beschrieben erfolgen.

Als Quellen für die elementaren Konstituenten eignen sich im Rahmen der Durchführung der vorstehend beschriebenen Herstellweise der katalytisch aktiven Oxidmassen der allgemeinen Formel (I) die oben bei der Multimetalloxidmasse (I) beschriebenen Ausgangsverbindungen oder Quellen.

Besonders bevorzugt sind Schalenkatalysatoren, die als katalytisch aktive Oxidmasse der allgemeinen Formel (I) die Multimetalloxidmasse (II) aufweisen.

Zur Herstellung der Schalenkatalysatoren eignen sich aber auch die aktiven Oxidmassen der allgemeinen Formel (I) aus der WO 00/29106, die im wesentlichen eine amorphe Struktur aufweisen, die im Röntgendiffraktogramm in Form von sehr breiten Beugungsreflexen mit Scheitelpunkten im Beugungswinkel - (2θ) - Bereich um 22° und um 27° abgebildet wird.

Geeignet sind aber auch die aktiven Oxidmassen der allgemeinen Formel (I) aus der EP-A-0 529 853 und aus der EP-A-0 608 838, die im Röntgendiffraktogramm sehr schmale Beugungsreflexe bei 2θ-Scheitelpunkt-Lagen von $22,1 \pm 0,3°$, $28,2 \pm 0,3°$, $36,2 \pm 0,3°$, $45,2 \pm 0,3°$ und $50,0 \pm 0,3°$ aufweisen.

Die Herstellung der Schalenkatalysatoren kann nicht nur durch Aufbringen der fertiggestellten, feingemahlenen aktiven Oxidmassen der allgemeinen Formel (I) auf die befeuchtete Trägerkörperoberfläche erfolgen, sondern anstelle der aktiven Oxidmasse kann auch eine feinteilige Vorläufermasse derselben auf die befeuchtete Trägeroberfläche (unter Anwendung der gleichen Beschichtungsverfahren und Bindemittel) aufgebracht und die Calcination nach Trocknung des beschichteten Trägerkörpers durchgeführt werden. Als eine solche feinteilige Vorläufermasse kommt zum Beispiel diejenige Masse in Betracht, die dadurch erhältlich ist, dass man aus den Quellen der elementaren Konstituenten der gewünschten aktiven Oxidmasse der allgemeinen Formel (I) zunächst ein möglichst inniges, vorzugsweise feinteiliges, Trockengemisch erzeugt (zum Beispiel durch Sprühtrocknung einer wäßrigen Suspension oder Lösung der Quellen) und dieses feinteilige Trockengemisch (gegebenenfalls nach Tablettierung unter Zusatz von 0,5 bis 2 Gew.-% an feinteiligem Graphit) bei einer Temperatur von 150 bis 350°C, vorzugsweise 250 bis 350°C unter oxidierender (Sauerstoff enthaltender) Atmosphäre (zum Beispiel unter Luft) thermisch behandelt (wenige Stunden) und abschließend bei Bedarf einer Mahlung unterwirft. Nach der Beschichtung der Trägerkörper mit der Vorläufermasse wird dann, bevorzugt unter Inertgasatmosphäre (alle anderen Atmosphären kommen auch in Betracht) bei Temperaturen von 360 bis 700°C beziehungsweise 400 bis 650°C oder 400 bis 600°C calciniert.

Die oben beschriebene Multimetalloxidmassen (II) oder die Katalysatoren (III) mit der Multimetalloxidmasse (II) als der katalytisch aktiven Oxidmasse können ebenfalls für die Oxidation von Propen verwendet werden. Hierbei kann das Propen im Beisein von Propan oxidiert werden. Wird Propan als Verdünnungsgas verwendet, kann es teilweise ebenfalls zu Acrylsäure oxidiert werden.

Die Durchführung der Propanoxidation unterliegt keinen Beschränkungen. Sie kann gemäß allen dem Fachmann bekannten Verfahren durchgeführt werden. Beispielsweise kann gemäß der in EP-A-0 608 838 oder WO 00/29106 beschriebenen Verfahrensweise gearbeitet werden. Das heißt, ein Gas B, mit dem der Katalysator bei Reaktionstemperaturen von zum Beispiel 200 bis 550°C oder von 230 bis 480°C beziehungsweise 300 bis 440°C in Schritt (d) zu belasten ist, kann zum Beispiel nachfolgende Zusammensetzung aufweisen:

1 bis 15, vorzugsweise 1 bis 7 Vol.% Propan,
44 bis 99 Vol.-% Luft und
0 bis 55 Vol.-% Wasserdampf.

Als andere mögliche Zusammensetzungen des in Schritt (d) geführten Gasgemisches zur Erzeugung des Gases B kommen in Betracht:

70 bis 95 Vol.-% Propan,
5 bis 30 Vol.-% molekularer Sauerstoff und
0 bis 25 Vol.-% Wasserdampf.

Zur Durchführung der Propanoxidation eignen sich auch die in der DE-A 199 52 964 beschriebenen Plattenwärmetauscherreaktoren. Ebenso kann eine Propanoxidation gemäß den in den DE-A 198 37 517, DE-A 198 37 518, DE-A 198 37 519 und DE-A 198 37 520 beschriebenen Verfahren durchgeführt werden.

Das die Propenoxidation als Schritt (d) des erfindungsgemäßen Verfahrens verlassende Produktgasgemisch besteht nicht ausschließlich aus dem Zielprodukt Acrolein und/oder Acrylsäure, sondern ist in der Regel im wesentlichen zusammengesetzt aus Acrolein und/oder Acrylsäure, nicht umgesetztem molekularen Sauerstoff, Propan, Propen, molekularem Stickstoff, als Nebenprodukt entstandenem und/oder als Verdünnungsgas mitverwendetem Wasserdampf, als Nebenprodukt und/oder als Verdünnungsgas mitverwendeten Kohlenoxiden, sowie geringen Mengen sonstiger niederer Aldehyde, Kohlenwasserstoffe und anderer inerter Verdünnungsgase.

Das Zielprodukt Acrolein und/oder Acrylsäure kann aus dem Produktgasgemisch in an sich bekannter Weise abgetrennt werden, beispielsweise über azeotrope Trennung, fraktionierte Destillation (mit oder ohne Lösungsmittel) oder Kristallisation. Beispielsweise geeignet sind eine partielle Kondensation der Acrylsäure, eine Absorption von Acrylsäure in Wasser oder in ein hochsiedendes hydrophobes organisches Lösungsmittel oder eine Absorption von Acrolein in Wasser oder in wäßrigen Lösungen niederer Carbonsäuren mit anschließender Aufarbeitung der Absorbate; alternativ kann das Produktgasgemisch auch fraktioniert kondensiert werden, vergleiche zum Beispiel EP-A-0 117 146, DE-A 43 08 087, DE-A 43 35 172, DE-A 44 36 243, DE-A 199 24 532 sowie DE-A 199 24 533.

In einer besonders bevorzugten Ausgestaltung des erfindungsgemäßen Verfahrens werden nach Durchführung von Schritt (d) nicht umgesetztes Propan und/oder Propen nach Abtrennung des Zielproduktes von dem dann verbleibenden Gasgemisch erfindungsgemäß gemäß Schritt (b) und (c) abgetrennt und wieder in Schritt (d) zurückgeführt. Bei dem in Schritt (b) des erfindungsgemäßen Verfahrens eingesetzten Gasgemisch A handelt es sich auch um ein Gasgemisch, das die Zusammensetzung eines Gasgemisches hat, das durch katalytische Dehydrierung von Propan zu Propen erhältlich ist. Hierbei erfolgt die Dehydrierung oxidativ, das heißt mit Sauerstoffzufuhr, oder ohne Sauerstoffzufuhr, insbesondere im wesentlichen ohne Sauerstoffzufuhr. Bei Dehydrierung mit Sauerstoffzufuhr lassen sich zwei Fälle unterscheiden. In einem Fall wird sämtlicher gebildeter Wasserstoff durch einen Überschuß an Sauerstoff oxidiert, so daß das Produktgas keinen Wasserstoff mehr, jedoch überschüssigen Sauerstoff enthält (oxidative Dehydrierung). Im zweiten Fall wird nur soviel Sauerstoff zugegeben, um die Reaktionsenthalpie zu decken, so daß im Produktgas kein Sauerstoff, wohl aber Wasserstoff enthalten ist (autotherme Fahrweise). Die Propandehydrierung kann katalytisch oder homogen (nicht katalytisch) durchgeführt werden.

Eine Dehydrierung von Propan gemäß Schritt (a) kann beispielsweise durchgeführt werden wie in der DE-A 33 13 573 und EP-A-0 117 146 beschrieben.

Prinzipiell kann die oxidative Propandehydrierung als homogene und/oder heterogen katalysierte Oxidehydrierung von Propan zu Propen mit molekularem Sauerstoff durchgeführt werden.

Gestaltet man diese erste Reaktionsstufe als eine homogene Oxidehydrierung, so läßt sich diese prinzipiell durchführen wie beschrieben zum Beispiel in den Schriften US-A-3 798 283, CN-A-1 105 352, Applied Catalysis, 70(2) 1991, S. 175-187, Catalysis Today 13, 1992, S. 673-678 und der Anmeldung DE-A-196 22 331, wobei als Sauerstoffquelle auch Luft eingesetzt werden kann.

Die Temperatur der homogenen Oxidehydrierung wird zweckmäßigerweise im Bereich von 300 bis 700°C, vorzugsweise im Bereich von 400 bis 600°C, besonders bevorzugt im Bereich von 400 bis 500°C liegend gewählt. Der Arbeitsdruck kann 0,5 bis 100 bar, insbesondere 1 bis 10 bar betragen. Die Verweilzeit liegt üblicherweise bei 0,1 beziehungsweise 0,5 bis 20 Sekunden, vorzugsweise bei 0,1 beziehungsweise 0,5 bis 5 Sekunden. Als Reaktor kann zum Beispiel ein Rohrofen oder ein Rohrbündelreaktor verwendet werden, wie zum Beispiel ein Gegenstrom-Rohrofen mit Rauchgas als Wärmeträger oder ein Rohrbündelreaktor mit Salzschmelze als Wärmeträger. Das Propan zu Sauerstoff-Verhältnis im Ausgangsgemisch beträgt vorzugsweise 0,5:1 bis 40:1, insbesondere zwischen 1:1 bis 6:1, stärker bevorzugt zwischen 2:1 bis 5:1. Das Ausgangsgemisch kann auch weitere, im wesentlichen inerte, Bestandteile, wie Wasser, Kohlendioxid, Kohlenmonoxid, Stickstoff, Edelgase und/oder Propen umfassen, wobei es sich hierbei auch um zurückgeführte Bestandteile handeln kann. Vorliegend werden in die Stufe (b) zurückgeführte Komponenten allgemein als Kreisgas bezeichnet.

Gestaltet man die Propandehydrierung als eine heterogen katalysierte Oxidehydrierung, so läßt sich diese prinzipiell durchführen wie beschrieben zum Beispiel in den Schriften US-A 4 788 371, CN-A 1073893, Catalysis Letters 23 (1994), 103-106, W. Zhang, Gaodeng Xuexiao Huaxue Xuebao, 14 (1993) 566, Z. Huang, Shiyou Huagong, 21 (1992) 592, WO 97/36849, DE-A 197 53 817, US-A 3 862 256, US-A 3 887 631, DE-A 195 30 454, US-A 4 341 664, J. of Catalysis 167, 560-569 (1997), J. of Catalysis 167, 550-559 (1997), Topics in Catalysis 3 (1996) 265-275, US-A 5 086 032, Catalysis Letters 10 (1991), 181-192, Ind. Eng. Chem. Res. 1996, 35, 14-18, US-A 4 255 284, Applied Catalysis A: General, 100 (1993), 111-130, J. of Catalysis 148, 56-67 (1994), V. Cortés Corberán und S. Vic Bellón (Ed.), New Developments in Selective Oxidation II, 1994, Elsevier Science B.V., S. 305-313, 3rd World Congress on Oxidation Catalysis, R.K. Grasselli, S.T. Oyama, A.M. Gaffney and J.E. Lyons (Ed.), 1997, Elsevier Science B.V., S. 375 ff.. Dabei kann als Sauerstoffquelle auch Luft eingesetzt werden. Vorzugsweise weist jedoch die Sauerstoffquelle mindestens 90 Mol-% Sauerstoff, stärker bevorzugt 95 Mol-% Sauerstoff, bezogen auf 100 Mol-% Sauerstoffquelle, auf.

Die für die heterogene Oxidehydrierung geeigneten Katalysatoren unterliegen keinen besonderen Beschränkungen. Es eignen sich alle dem Fachmann auf diesem Gebiet bekannten Oxidehydrierungskatalysatoren, die in der Lage sind, Propan zu Propen zu oxidieren. Insbesondere können alle in den zuvor genannten Schriften genannten Oxidehydrierungskatalysatoren eingesetzt werden. Bevorzugte Katalysatoren umfassen beispielsweise Oxidehydrierungskatalysatoren, die MoVNb-Oxide oder Vanadylpyrophosphat, jeweils mit Promotor, umfassen. Ein Beispiel für einen besonders gut geeigneten Katalysator ist ein Katalysator, der ein Mischmetalloxid mit Mo, V, Te, O und X als wesentlichen Bestandteile enthält, wobei X mindestens ein Element ist, ausgewählt aus Niob, Tantal, Wolfram, Titan, Aluminium, Zirkonium, Chrom, Mangan, Eisen, Ruthenium, Kobalt, Rhodium, Nickel, Palladium, Platin, Antimon, Bismut, Bor, Indium und Cer. Weiterhin besonders geeignete Oxidehydrierungskatalysatoren sind die Multimetalloxidmassen beziehungsweise -katalysatoren A der DE-A-197 53 817, wobei die in der vorgenannten Schrift als bevorzugt genannten Multimetalloxidmassen beziehungsweise -katalysatoren A ganz besonders günstig sind. Das heißt, als Aktivmassen kommen insbesondere Multimetalloxidmassen (IV) der allgemeinen Formel IV

$$M^1_a MO_{1-b} M^2_b O_x \qquad (IV),$$

wobei

$M^1$ = Co, Ni, Mg, Zn, Mn und/oder Cu,

$M^2$ = W, V, Te, Nb, P, Cr, Fe, Sb, Ce, Sn und/oder La,

a = 0,5-1,5

b = 0-0,5

sowie

x = eine Zahl, die durch die Wertigkeit und Häufigkeit der von Sauerstoff verschiedenen Elemente in (IV) bestimmt wird,

in Betracht.

Prinzipiell können geeignete Aktivmassen (IV) in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 450 bis 1000°C calciniert. Als Quellen für die elementaren Konstituenten der Multimetalloxid-Aktivmassen (IV) kommen solche Verbindungen in Betracht, bei denen es sich bereits um Oxide handelt und/oder solche Verbindungen, die durch Erhitzen, wenigstens in Anwesenheit von Sauerstoff, in Oxide überführbar sind. Hierbei handelt es sich vom allem um Halogenide, Nitrate, Formiate, Oxalate, Citrate, Acetate, Carbonate, Amminkomplexsalze, Ammoniumsalze und/oder Hydroxide. Das innige Vermischen der Ausgangsverbindungen zur Herstellung der Multimetalloxidmassen (IV) kann in trockener Form, zum Beispiel als feinteiliges Pulver, oder in nasser Form, zum Beispiel mit Wasser als Lösungmittel, erfolgen. Die Multimetalloxidmassen (IV) können sowohl in Pulverform als auch zu bestimmten Katalysatorgeometrien geformt, eingesetzt werden, wobei die Formgebung vor oder nach der abschließenden Calcinierung erfolgen kann. Es können Vollkatalysatoren eingesetzt werden oder aber die Formgebung einer pulverförmigen Aktivmasse beziehungsweise Vorläufermasse kann auch durch Aufbringung auf vorgeformte inerte Katalysatorträger erfolgen. Als Trägermaterialien können dabei übliche, poröse oder unporöse Aluminiumoxide, Siliciumdioxid, Thoriumdioxid, Zirkondioxid, Siliciumcarbid oder Silikate verwendet werden, wobei die Trägerkörper regelmäßig oder unregelmäßig geformt sein können.

Für die heterogen katalysierte Oxidehydrierung des Propans liegt die Reaktionstemperatur vorzugsweise im Bereich von 200 bis 600°C, insbesondere im Bereich von 250 bis 500°C, stärker bevorzugt im Bereich von 350 bis 440°C. Der Arbeitsdruck liegt vorzugsweise im Bereich von 0,5 bis 10 bar, insbesondere von 1 bis 10 bar, stärker bevorzugt von 1 bis 5 bar. Arbeitsdrucke oberhalb von 1 bar, zum Beispiel von 1,5 bis 10 bar, haben sich als besonders vorteilhaft erwiesen. In der Regel erfolgt die heterogen katalysierte Oxidehydrierung des Propans an einem Katalysatorfestbett. Letzteres wird zweckmäßigerweise in den Rohren eines Rohrbündelreaktors aufgeschüttet, wie sie zum Beispiel in der EP-A-0 700 893 und in der EP-A-0 700 714 sowie der in diesen Schriften zitierten Literatur beschrieben sind. Die mittlere Verweilzeit des Reaktionsgasgemisches in der Katalysatorschüttung liegt zweckmäßigerweise bei 0,5 bis 20 Sekunden. Das Verhältnis von Propan zu Sauerstoff variiert mit dem gewünschten Umsatz und der Selektivität des Katalysators, zweckmäßigerweise liegt es im Bereich von 0,5:1 bis 40:1, insbesondere von 1:1 bis 6:1, stärker bevorzugt von 2:1 bis 5:1. In der Regel nimmt die Propenselektivität mit steigendem Propanumsatz ab. Vorzugsweise wird deshalb die Propan zu Propen-Reaktion so durchgeführt, daß relativ niedrige Umsätze mit Propan bei hohen Selektivitäten zu Propen erreicht werden. Besonders bevorzugt liegt der Umsatz an Propan im Bereich von 5 bis 40%, stärker bevorzugt von 10 bis 30%. Hierbei bedeutet der Begriff "Propanumsatz" den Anteil an zugeführtem Propan, das umgesetzt wird. Besonders bevorzugt beträgt die Selektivität 50 bis 98%, stärker bevorzugt 80 bis 98%, wobei der Begriff "Selektivität" die Mole an Propen bezeichnet, die pro Mol umgesetztem Propan erzeugt werden, ausgedrückt als Prozentsatz.

Vorzugsweise enthält das bei der oxidativen Propandehydrierung eingesetzte Ausgangsgemisch 5 bis 95 Gew.

% Propan, bezogen auf 100 Gew.-% Ausgangsgemisch. Neben Propan und Sauerstoff kann das Ausgangsgemisch für die heterogen katalysierte Oxidehydrierung auch weitere, insbesondere inerte, Bestandteile wie Wasser, Kohlendioxid, Kohlenmonoxid, Stickstoff, Edelgase und/oder Propen umfassen. Die heterogene Oxidehydrierung kann auch in Anwesenheit von Verdünnungsmitteln, wie zum Beispiel Dampf, durchgeführt werden.

Jede beliebige Reaktorsequenz kann zur Durchführung der homogenen Oxidehydrierung oder der heterogen katalysierten Oxidehydrierung eingesetzt werden, die dem Fachmann bekannt ist. Zum Beispiel kann die Reaktion in einer einzigen Stufe oder in zwei oder mehreren Stufen, zwischen denen Sauerstoff eingeführt wird, durchgeführt werden. Es besteht auch die Möglichkeit, homogene und heterogen katalytische Oxidehydrierung miteinander kombiniert zu verwenden.

Als mögliche Bestandteile kann das Produktgemisch der Propanoxidehydrierung zum Beispiel folgende Komponenten enthalten: Propen, Propan, Kohlendioxid, Kohlenmonoxid, Wasser, Stickstoff, Sauerstoff, Ethan, Ethen, Methan, Acrolein, Acrylsäure, Ethylenoxid, Butan, Essigsäure, Formaldehyd, Ameisensäure, Propylenoxid und Buten. Ein bevorzugtes, bei der Propanoxidehydrierung erhaltenes Produktgemisch enthält: 5 bis 10 Gew.-% Propen, 1 bis 2 Gew.-% Kohlenmonoxid, 1 bis 3 Gew.-% Kohlendioxid, 4 bis 10 Gew.-% Wasser, 0 bis 1 Gew.-% Stickstoff, 0,1 bis 0,5 Gew.% Acrolein, 0 bis 1 Gew.-% Acrylsäure, 0,05 bis 0,2 Gew.-% Essigsäure, 0,01 bis 0,05 Gew.-% Formaldehyd, 1 bis 5 Gew.-% Sauerstoff, 0,1 bis 1,0 Gew.-% weitere oben genannte Komponenten, sowie Rest Propan, jeweils bezogen auf 100 Gew.-% Produktgemisch.

Da die Dehydrierreaktion unter Volumenzunahme abläuft, kann der Umsatz durch Erniedrigung des Partialdrucks der Produkte gesteigert werden. Dies läßt sich in einfacher Weise, zum Beispiel durch Dehydrierung bei vermindertem Druck und/oder durch Zumischen von im wesentlichen inerten Verdünnungsgasen, wie zum Beispiel Wasserdampf, erreichen, der für die Dehydrierreaktion im Normalfall ein Inertgas darstellt. Eine Verdünnung mit Wasserdampf bedingt als weiteren Vorteil in der Regel ein vermindertes Verkoken des verwendeten Katalysators, da der Wasserdampf mit gebildetem Koks nach dem Prinzip der Kohlevergasung reagiert. Außerdem kann Wasserdampf als Verdünnungsgas in dem nachfolgenden Oxidationsschritt (d) mitverwendet werden. Weitere für die Propandehydrierung geeignete Verdünnungsmittel sind zum Beispiel CO, $CO_2$, Stickstoff und Edelgase wie He, Ne und Ar. Alle genannten Verdünnungsmittel können entweder für sich oder in Form unterschiedlichster Gemische mitverwendet werden. Es ist von Vorteil, dass die genannten Verdünnungsmittel in der Regel auch für den Oxidationsschritt (d) geeignete Verdünnungsmittel sind. Generell sind sich in der jeweiligen Stufe inert verhaltende (das heißt zu weniger als 5 mol-%, bevorzugt zu weniger als 3 mol-% und noch besser zu weniger als 1 mol-% sich chemisch verändernde) Verdünnungsmittel bevorzugt. Prinzipiell kommen für die Propandehydrierung alle im Stand der Technik bekannten Dehydrierkatalysatoren in Betracht. Sie lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen, Träger abgeschiedenen, in der Regel vergleichsweise edlen, Metall (zum Beispiel Platin) bestehen.

Unter anderem können damit alle Dehydrierkatalysatoren eingesetzt werden, die in der WO 99/46039, der US-A 4 788 371, der EP-A-0 705 136, der WO 99/29420, der US-A 4 220 091, der US-A 5 430 220, der US-A 5 877 369, der EP-A-0 117 146, der DE-A 199 37 196, der DE-A 199 37 105 sowie der DE-A 199 37 107 empfohlen werden. Im besonderen können sowohl der Katalysator gemäß Beispiel 1, Beispiel 2, Beispiel 3, und Beispiel 4 der DE-A 199 37 107 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

Zur Durchführung der Propandehydrierung kommen prinzipiell alle im Stand der Technik bekannten Reaktortypen und Verfahrensvarianten in Betracht. Beschreibungen solcher Verfahrensvarianten enthalten zum Beispiel alle bezüglich der Dehydrierkatalysatoren genannten Schriften des Standes der Technik.

Eine vergleichsweise ausführliche Beschreibung von erfindungsgemäß geeigneten Dehydrierverfahren enthält auch "Catalytical Studies Division, Oxidative Dehydrogenation and Alternative Dehydrogenation Processes", Study Number 4192 OD, 1993, 430 Ferguson Drive, Mountain View, California, 94043-5272 U.S.A..

Charakteristisch für die partielle heterogen katalysierte Dehydrierung von Propan ist, dass sie endotherm verläuft. Das heißt, die für die Einstellung der erforderlichen Reaktionstemperatur benötigte Wärme (Energie) muß entweder dem Reaktionsgas vorab und/oder im Verlauf der katalytischen Dehydrierung zugeführt werden.

Ferner ist es für heterogen katalysierte Dehydrierungen von Propan aufgrund der hohen benötigten Reaktionstemperaturen typisch, dass in geringen Mengen schwersiedende hochmolekulare organische Verbindungen, bis hin zum Kohlenstoff, gebildet werden, die sich auf der Katalysatoroberfläche abscheiden und selbige dadurch deaktivieren. Um diese nachteilige Begleiterscheinung zu minimieren, kann, das zur katalytischen Dehydrierung bei erhöhter Temperatur über die Katalysatoroberfläche zu leitende Propan mit Wasserdampf verdünnt werden. Sich abscheidender Kohlenstoff wird unter den so gegebenen Bedingungen nach dem Prinzip der Kohlevergasung teilweise oder vollständig eliminiert.

Eine andere Möglichkeit, abgeschiedene Kohlenstoffverbindungen zu beseitigen, besteht darin, den Dehydrierkatalysator von Zeit zu Zeit bei erhöhter Temperatur mit einem Sauerstoff enthaltenden Gas zu durchströmen und damit den abgeschiedenen Kohlenstoff quasi abzubrennen. Eine Unterdrückung der Bildung von Kohlenstoffablagerungen ist aber auch dadurch möglich, dass man dem katalytisch zu dehydrierenden Propan, bevor es bei erhöhter Temperatur über den Dehydrierkatalysator geführt wird, molekularen Wasserstoff zusetzt

Selbstverständlich besteht auch die Möglichkeit, Wasserdampf und molekularen Wasserstoff dem katalytisch zu dehydrierenden Propan im Gemisch zuzusetzen. Ein Zusatz von molekularem Wasserstoff zur katalytischen Dehydrierung von Propan mindert auch die unerwünschte Bildung von Allen und Acetylen als Nebenprodukten.

Eine geeignete Reaktorform für die Propandehydrierung ist der Festbettrohrbeziehungsweise Rohrbündelreaktor. Das heißt, der Dehydrierkatalysator befindet sich in einem oder in einem Bündel von Reaktionsrohren als Festbett. Die Reaktionsrohre werden dadurch beheizt, dass im die Reaktionsrohre umgebenden Raum ein Gas, zum Beispiel ein Kohlenwasserstoff wie Methan, verbrannt wird. Günstig ist es, diese direkte Form der Kontaktrohrerwärmung lediglich auf den ersten etwa 20 bis 30% der Festbettschüttung anzuwenden und die verbleibende Schüttungslänge durch die im Rahmen der Verbrennung freigesetzte Strahlungswärme auf die erforderliche Reaktionstemperatur aufzuwärmen. Auf diesem Weg ist eine annähernd isotherme Reaktionsfiihrung erreichbar. Geeignete Reaktionsrohrinnendurchmesser betragen etwa 10 bis 15 cm. Ein typischer Dehydrierrohrbündelreaktor umfaßt 300 bis 1000 Reaktionsrohre. Die Temperatur im Reaktionsrohrinneren bewegt sich im Bereich von 300 bis 700°C, vorzugsweise im Bereich von 400 bis 700°C. Mit Vorteil wird das Reaktionsgas dem Rohrreaktor auf die Reaktionstemperatur vorerwärmt zugeführt. Häufig verläßt das Produktgasgemisch das Reaktionsrohr mit einer 50 bis 100°C tiefergelegenen Temperatur. Im Rahmen der vorgenannten Verfahrensweise ist die Verwendung von oxidischen Dehydrierkatalysatoren auf der Grundlage von Chrom- und/oder Aluminiumoxid zweckmäßig. Häufig wird man kein Verdünnungsgas mitverwenden, sondern von im wesentlichen reinem Propan als Ausgangsreaktionsgas ausgehen. Auch der Dehydrierkatalysator wird meist unverdünnt angewandt.

Großtechnisch würde man etwa drei Rohrbündelreaktoren parallel betreiben. Zwei dieser Reaktoren würden sich in der Regel im Dehydrierbetrieb befinden, während in einem der Reaktoren die Katalysatorbeschickung regeneriert wird.

Vorstehende Verfahrensweise wird beispielsweise beim in der Literatur bekannten BASF-Linde Propan-Dehydrierverfahren angewendet.

Des weiteren wird sie beim sog. "steam active reforming (STAR) process" angewendet, der von der Phillips Petroleum Co. entwickelt wurde (vergleiche zum Beispiel US-A 4 902 849, US-A 4 996 387 und US-A 5 389 342). Als Dehydrierkatalysator wird im STAR-Prozess mit Vorteil Promotoren enthaltendes Platin auf Zink (Magnesium) Spinel als Träger angewendet (vergleiche zum Beispiel US-A 5 073 662). Im Unterschied zum BASF-Linde Propan-Dehydrierverfahren wird das zu dehydrierende Propan beim STAR-Prozess mit Wasserdampf verdünnt. Typisch ist ein molares Verhältnis von Wasserdampf zu Propan im Bereich von 4 bis 6. Der Arbeitsdruck liegt häufig bei 3 bis 8 atm und die Reaktionstemperatur wird zweckmäßig zu 480 bis 620°C gewählt. Typische Katalysatorbelastungen mit dem totalen Reaktionsgasgemisch liegen bei 0,5 bis 10 $h^{-1}$.

Die Propandehydrierung kann auch im Wanderbett gestaltet werden. Beispielsweise kann das Katalysatorwanderbett in einem Radialstromreaktor untergebracht sein. In selbigem bewegt sich der Katalysator langsam von oben nach unten, während das Reaktionsgasgemisch radial fließt. Diese Verfahrensweise wird beispielsweise im sogenannten UOP-Oleflex-Dehydrierverfahren angewandt. Da die Reaktoren bei diesem Verfahren quasi adiabat betrieben werden, ist es zweckmäßig, mehrere Reaktoren hintereinander geschaltet zu betreiben (in typischer Weise bis zu vier). Dadurch lassen sich zu hohe Unterschiede der Temperaturen des Reaktionsgasgemisches am Reaktoreingang und am Reaktorausgang vermeiden (bei der adiabaten Betriebsweise fungiert das Reaktionsgasausgangsgemisch als Wärmeträger, von dessen Wärmeinhalt die Reaktionstemperatur abhängig ist) und trotzdem ansprechende Gesamtumsätze erzielen.

Wenn das Katalysatorbett den Wanderbettreaktor verlassen hat, wird es der Regenerierung zugeführt und anschließend wiederverwendet. Als Dehydrierkatalysator kann für dieses Verfahren zum Beispiel ein kugelförmiger Dehydrierkatalysator eingesetzt werden, der im wesentlichen aus Platin auf kugelförmigem Aluminiumoxidträger besteht. Bei der UOP-Variante wird dem zu dehydrierenden Propan Wasserstoff zugefügt, um eine vorzeitige Katalysatoralterung zu vermeiden. Der Arbeitsdruck liegt typisch bei 2 bis 5 atm. Das Wasserstoff zu Propan-Verhältnis (das molare) beträgt zweckmäßig 0,1 bis 1. Die Reaktionstemperaturen liegen bevorzugt bei 550 bis 650°C und die Kontaktzeit des Katalysators mit Reaktionsgasgemisch wird zu etwa 2 bis 6 $h^{-1}$ gewählt.

Bei den beschriebenen Festbettverfahren kann die Katalysatorgeometrie ebenfalls kugelförmig, aber auch zylindrisch (hohl oder voll) sein.

Als weitere Verfahrensvariante für die Propandehydrierung beschreibt Proceedings De Witt, Petrochem. Review, Houston, Texas, 1992 a, N1 die Möglichkeit einer heterogen katalysierten Propandehydrierung im Wirbelbett, bei der das Propan nicht verdünnt wird.

Zweckmäßigerweise werden dabei zwei Wirbelbetten nebeneinander betrieben, von denen sich eines in der Regel im Zustand der Regenerierung befindet. Als Aktivmasse kommt dabei Chromoxid auf Aluminiumoxid zum Einsatz. Der Arbeitsdruck beträgt typisch 1 bis 1,5 atm und die Dehydriertemperatur liegt in der Regel bei 550 bis 600°C. Die für die Dehydrierung erforderliche Wärme wird dadurch in das Reaktionssystem eingebracht, dass der Dehydrierkatalysator auf die Reaktionstemperatur vorerhitzt wird. Der Arbeitsdruck liegt regelmäßig bei 1 bis 2 atm und die Reaktionstemperatur beträgt typisch 550 bis 600°C. Die vorstehende Dehydrierweise ist in der Literatur auch als Snamprogetti-Yarsintez Verfahren bekannt.

Den bisher beschriebenen heterogen katalysierten Dehydrierverfahren des Propans unter im wesentlichen Sauerstoffausschluß ist gemein, dass sie bei Propanumsätzen von > 30 mol-% (in der Regel ≤ 60 mol-%) betrieben werden (bezogen auf einmaligen Reaktordurchgang). Von Vorteil ist es, dass es ausreichend ist, einen Propanumsatz von ≥ 5 mol-% bis ≤ 30 mol-% oder ≤ 25 mol-%, zu erzielen. Das heißt, die Propandehydrierung kann auch bei Propanumsätzen von 10 bis 20 mol-% betrieben werden (die Umsätze beziehen sich auf einmaligen Reaktordurchgang). Dies rührt unter anderem daher, dass die verbliebene Menge an nicht umgesetztem Propan im nachfolgenden Oxidationsschritt (d) mit molekularem Stickstoff.verdünnt wird, was die Propionaldehyd- und/oder Propionsäurenebenproduktbildung mindert.

Für die Realisierung der vorgenannten Propanumsätze ist es günstig, die Propandehydrierung bei einem Arbeitsdruck von 0,3 bis 3 atm durchzuführen. Ferner ist es günstig, das zu dehydrierende Propan mit Wasserdampf zu verdünnen. So ermöglicht die Wärmekapazität des Wassers einerseits, einen Teil der Auswirkung der Endothermie der Dehydrierung auszugleichen und andererseits reduziert die Verdünnung mit Wasserdampf den Edukt- und Produktpartialdruck, was sich günstig auf die Gleichgewichtslage der Dehydrierung auswirkt. Ferner wirkt sich die Wasserdampfmitverwendung, wie bereits erwähnt, vorteilhaft auf die Standzeit des Dehydrierkatalysators aus. Bei Bedarf kann als weiterer Bestandteil auch molekularer Wasserstoff zugegeben werden. Das molare Verhältnis von molekularem Wasserstoff zu Propan ist dabei in der Regel ≤ 5. Das molare Verhältnis von Wasserdampf zu Propan kann demnach mit vergleichsweise geringem Propanumsatz ≥ 0 bis 30, zweckmäßig 0,1 bis 2 und günstig 0,5 bis 1 betragen. Als günstig für eine Verfahrensweise mit niederem Propanumsatz erweist es sich auch, dass bei einmaligem Reaktordurchgang des Reaktionsgases lediglich eine vergleichsweise niedrige Wärmemenge verbraucht wird und zur Umsatzerzielung bei einmaligem Reaktordurchgang vergleichsweise niedrige Reaktionstemperaturen ausreichend sind.

Es kann daher zweckmäßig sein mit vergleichsweise geringem Propanumsatz die Propandehydrierung (quasi) adiabat durchzuführen. Das heißt, man wird das Reaktionsgasausgangsgemisch in der Regel auf eine Temperatur von 500 bis 700°C erhitzen (zum Beispiel durch Direktbefeuerung der es umgebenden Wandung), beziehungsweise auf 550 bis 650°C. Im Normalfall wird dann ein einziger adiabater Durchgang durch ein Katalysatorbett ausreichend sein, um den gewünschten Umsatz zu erzielen, wobei sich das Reaktionsgasgemisch um etwa 30°C bis 200°C (je nach Umsatz) abkühlen wird. Ein Beisein von Wasserdampf als Wärmeträger macht sich auch unter dem Gesichtspunkt einer adiabaten Fahrweise vorteilhaft bemerkbar. Die niedrigere Reaktionstemperatur ermöglicht längere Standzeiten des verwendeten Katalysatorbetts.

Prinzipiell ist die Propandehydrierung mit vergleichsweise geringem Propanumsatz, ob adiabat oder isotherm gefahren, sowohl in einem Festbettreaktor als auch in einem Wanderbett- oder Wirbelbettreaktor durchführbar.

Bemerkenswerterweise ist zu ihrer Realisierung, insbesondere im adiabatischen Betrieb, ein einzelner Schachtofenreaktor als Festbettreaktor ausreichend, der vom Reaktionsgasgemisch axial und/oder radial durchströmt wird.

Im einfachsten Fall handelt es sich dabei um ein einziges geschlossenes Reaktionsvolumen, zum Beispiel ein Behälter, dessen Innendurchmesser 0,1 bis 10 m, eventuell auch 0,5 bis 5 m beträgt und in welchem das Katalysatorfestbett auf einer Trägervorrichtung (zum Beispiel ein Gitterrost) aufgebracht ist. Das mit Katalysator beschickte Reaktionsvolumen, das im adiabaten Betrieb wärmeisoliert ist, wird dabei mit dem heißen, Propan enthaltenden, Reaktionsgas axial durchströmt. Die Katalysatorgeometrie kann dabei sowohl kugelförmig als auch ringförmig und strangförmig sein. Da in diesem Fall das Reaktionsvolumen durch einen sehr kostengünstigen Apparat zu realisieren ist, sind alle Katalysatorgeometrien, die einen besonders niedrigen Druckverlust aufweisen, zu bevorzugen. Das sind vor allem Katalysatorgeometrien, die zu einem großen Hohlraumvolumen führen oder strukturiert aufgebaut sind, wie zum Beispiel Waben. Zur Verwirklichung einer radialen Strömung des Propan enthaltenden Reaktionsgases kann der Reaktor zum Beispiel aus zwei in einer Mantelhülle befindlichen, zentrisch ineinander gestellten, zylindrischen Gitterrosten bestehen und die Katalysatorschüttung in deren Ringspalt angeordnet sein. Im adiabaten Fall wäre die Metallhülle wiederum thermisch isoliert.

Als Katalysatorbeschickung für eine Propandehydrierung mit vergleichsweise geringem Propanumsatz bei einmaligem Durchgang eignen sich insbesondere die in der DE-A 199 37 107 offenbarten, vor allem alle beispielhaft offenbarten, Katalysatoren.

Nach längerer Betriebsdauer sind vorgenannte Katalysatoren zum Beispiel in einfacher Weise dadurch regenerierbar, dass man bei einer Temperatur von 300 bis 600°C, häufig bei 400 bis 500°C, zunächst in ersten Regenerierungsstufen mit Stickstoff verdünnte Luft über das Katalysatorbett leitet. Die Katalysatorbelastung mit Regeneriergas kann dabei z.B. 50 bis 10000 h$^{-1}$ und der Sauerstoffgehalt des Regeneriergases 0,5 bis 20 Vol.% betragen.

In nachfolgenden weiteren Regenerierungsstufen kann unter ansonsten gleichen Regenerierbedingungen als Regeneriergas Luft verwendet werden. Anwendungstechnisch zweckmäßig empfiehlt es sich, den Katalysator vor seiner Regenerierung mit Inertgas (zum Beispiel $N_2$) zu spülen.

Anschließend ist es in der Regel empfehlenswert, noch mit reinem molekularen Wasserstoff oder mit durch Inertgas verdünntem molekularem Wasserstoff (der Wasserstoffgehalt sollte ≥ 1 Vol.-% betragen) im ansonsten gleichen Bedingungsraster zu regenerieren.

Die Propandehydrierung mit vergleichsweise geringem Propanumsatz (≤ 30 mol-%) kann in allen Fällen bei den gleichen Katalysatorbelastungen (sowohl das Reaktionsgas insgesamt, als auch das in selbigem enthaltende Propan betreffend) betrieben werden wie die Varianten mit hohem Propanumsatz (> 30 mol-%). Diese Belastung mit Reaktionsgas kann zum Beispiel 100 bis 10000 $h^{-1}$, häufig 100 bis 3000 $h^{-1}$, das heißt vielfach etwa 100 bis 2000 $h^{-1}$ betragen.

In besonders eleganter Weise läßt sich die Propandehydrierung mit vergleichsweise geringem Propanumsatz in einem Hordenreaktor verwirklichen.

Dieser enthält räumlich aufeinanderfolgend mehr als ein die Dehydrierung katalysierendes Katalysatorbett. Die Katalysatorbettanzahl kann 1 bis 20, zweckmäßig 2 bis 8, aber auch 3 bis 6 betragen. Die Katalysatorbetten sind vorzugsweise radial oder axial hintereinander angeordnet. Anwendungstechnisch zweckmäßig wird in einem solchen Hordenreaktor der Katalysatorfestbetttyp angewendet.

Im einfachsten Fall sind die Katalysatorfestbetten in einem Schachtofenreaktor axial oder in den Ringspalten von zentrisch ineinandergestellten zylindrischen Gitterrosten angeordnet. Es ist jedoch auch möglich, die Ringspalten in Segmenten übereinander anzuordnen und das Gas nach radialem Durchtritt in einem Segment in das nächste darüber- oder darunterliegende Segment zu führen.

In zweckmäßiger Weise wird das Reaktionsgasgemisch auf seinem Weg von einem Katalysatorbett zum nächsten Katalysatorbett, zum Beispiel durch Überleiten mit heißen Gasen erhitzte Wärmetauscherrippen oder durch Leiten durch mit heißen Brenngasen erhitzte Rohre, im Hordenreaktor einer Zwischenerhitzung unterworfen.

Wird der Hordenreaktor im übrigen adiabat betrieben, ist es für die gewünschten Propanumsätze (≤ 30 mol-%) insbesondere bei Verwendung der in der DE-A 199 37 107 beschriebenen Katalysatoren, insbesondere der beispielhaften Ausführungsformen, ausreichend, das Reaktionsgasgemisch auf eine Temperatur von 450 bis 550°C vorerhitzt in den Dehydrierreaktor zu führen und innerhalb des Hordenreaktors in diesem Temperaturbereich zu halten. Das heißt, die gesamte Propandehydrierung ist so bei äußerst niederen Temperaturen zu verwirklichen, was sich für die Standzeit der Katalysatorfestbetten zwischen zwei Regenerierungen als besonders günstig erweist.

Noch geschickter ist es, die vorstehend geschilderte Zwischenerhitzung auf direktem Weg durchzuführen (autotherme Fahrweise). Dazu wird dem Reaktionsgasgemisch entweder bereits vor Durchströmung des ersten Katalysatorbettes und/oder zwischen den nachfolgenden Katalysatorbetten in begrenztem Umfang molekularer Sauerstoff zugesetzt. Je nach verwendetem Dehydrierkatalysator wird so eine begrenzte Verbrennung der im Reaktionsgasgemisch enthaltenen Kohlenwasserstoffe, gegebenenfalls bereits auf der Katalysatoroberfläche abgeschiedener Kohle beziehungsweise kohleähnlicher Verbindungen und/oder von im Verlauf der Propandehydrierung gebildetem und/oder dem Reaktionsgasgemisch zugesetztem Wasserstoff bewirkt (es kann auch anwendungstechnisch zweckmäßig sein, im Hordenreaktor Katalysatorbetten einzufügen, die mit Katalysator beschickt sind, der spezifisch (selektiv) die Verbrennung von Wasserstoff (und/oder von Kohlenwasserstoff) katalysiert (als solche Katalysatoren kommen zum Beispiel jene der Schriften US-A 4 788 371, US-A 4 886 928, US-A 5 430 209, US-A 5 530 171, US-A 5 527 979 und US-A 5 563 314 in Betracht; beispielsweise können solche Katalysatorbetten in alternierender Weise zu den Dehydrierkatalysator enthaltenden Betten im Hordenreaktor untergebracht sein). Die dabei freigesetzte Reaktionswärme ermöglicht so auf quasi autotherme Weise eine nahezu isotherme Betriebsweise der heterogen katalysierten Propandehydrierung. Mit zunehmender gewählter Verweilzeit des Reaktionsgases im Katalysatorbett ist so eine Propandehydrierung bei abnehmender und im wesentlichen konstanter Temperatur möglich, was besonders lange Standzeiten zwischen zwei Regenerierungen ermöglicht.

In der Regel sollte eine wie vorstehend beschriebene Sauerstoffeinspeisung so vorgenommen werden, dass der Sauerstoffgehalt des Reaktionsgasgemisches, bezogen auf die darin enthaltene Menge an Propan und Propen, 0,5 bis 10 Vol.-% beträgt. Als Sauerstoffquelle kommen dabei sowohl reiner molekularer Sauerstoff oder mit Inertgas, zum Beispiel CO, $CO_2$, $N_2$, Edelgase, verdünnter Sauerstoff, insbesondere aber auch Luft, in Betracht. Die resultierenden Verbrennungsgase wirken in der Regel zusätzlich verdünnend und fördern dadurch die heterogen katalysierte Propandehydrierung.

Die Isothermie der heterogen katalysierten Propandehydrierung läßt sich dadurch weiter verbessern, dass man im Hordenreaktor in den Räumen zwischen den Katalysatorbetten geschlossene, vor ihrer Füllung günstigerweise aber nicht notwendigerweise evakuierte, Einbauten (zum Beispiel rohrförmige), anbringt. Derartige Einbauten können auch ins jeweilige Katalysatorbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Eine Möglichkeit, das Reaktionsgasgemisch in der Propandehydrierung auf die benötigte Reaktionstemperatur zu erwärmen, besteht auch darin, einen Teil des darin enthaltenen Propans und/oder $H_2$ mittels molekularem Sauerstoff

zu verbrennen (zum Beispiel an geeigneten spezifisch wirkenden Verbrennungskatalysatoren, zum Beispiel durch einfaches Überleiten und/oder Durchleiten) und mittels der so freigesetzten Verbrennungswärme die Erwärmung auf die gewünschte Reaktionstemperatur zu bewirken. Die resultierenden Verbrennungsprodukte, wie $CO_2$, $H_2O$ sowie das den für die Verbrennung benötigten molekularen Sauerstoff gegebenenfalls begleitende $N_2$ bilden vorteilhaft inerte Verdünnungsgase.

Erfindungsgemäß besteht auch die Möglichkeit, daß nach Durchführung von Schritt (d) und einer Abtrennung des Zielproduktes (Acrolein und/oder Acrylsäure) nicht umgesetztes Propan und gegebenenfalls Propen einer Dehydrierung von Propan unterzogen wird, die wie oben beschrieben durchgeführt werden kann, und das nach der Propandehydrierung erhaltene Produktgasgemisch wieder Schritt (b) unterworfen wird.

Allgemein bietet sich im Fall der Durchführung einer Propandehydrierung Propan in Schritt (d) als Verdünnungsgas an.

Es besteht auch die Möglichkeit, insbesondere bei Durchführung einer Propandehydrierung dem Schritt (d) zugeführtes Gas B noch reines Propan und/oder Propen zuzudosieren.

Wird der Schritt (d) als Umsetzung von Propen zu Acrylsäure durchgeführt, dann enthält das Abgas aus der Aufarbeitung neben den in der Oxidation nicht umgesetzten Komponenten Propan, Stickstoff und Rest-Sauerstoff noch oxidierbare Nebenkomponenten wie zum Beispiel Kohlenmonoxid, Ameisensäure, Formaldehyd, Essigsäure, sowie geringe Mengen an Acrylsäure. In einer besonders bevorzugten Ausführungsform werden diese Nebenkomponenten vor einer Propandehydrierung mit dem Rest-Sauerstoff und gegebenenfalls mit zusätzlichem molekularem Sauerstoff katalytisch oxidiert, um das Gas vor der Dehydrierung aufzuwärmen. Diese Oxidation könnte in einem Nachverbrennungskatalysator, wie zum Beispiel einem Pd-Katalysator auf einem Aluminiumoxidträger, beispielsweise RO-20/13 oder RO-20/25 (beide von BASF), erfolgen.

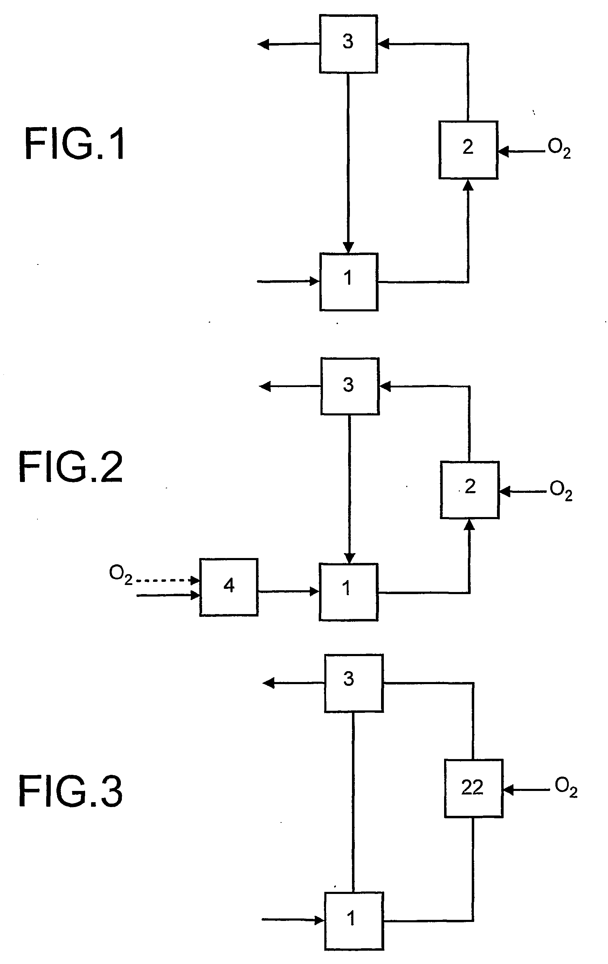

Bevorzugte Ausführungsformen der Erfindung sind in den nachstehend beschriebenen Figuren 1 2 4 5 und 6 gezeigt, die die Erfindung näher erläutern, ohne sie einzuschränken.

Fig. 1 bis 5 zeigen Schemaskizzen zur Durchführung von Verfahren zur Herstellung von Acrolein und/oder Acrylsäure, in denen zur Vereinfachung nicht alle Zu- und Ableitungsströme gezeigt sind. Fig. 1 zeigt eine Absorptions- und Desorptionstufe 1, eine Oxidationsstufe 2, die als Oxidation von Propen zu Acrolein und/oder Acrylsäure ausgebildet ist, und eine Aufarbeitungsstufe 3. In Fig. 1 wird in Absorptionsstufe 1 aus einem Gemisch, das Propan, Propen, Wasserstoff und Kohlenoxide (Kohlenmonoxid und Kohlendioxid) sowie eventuell Stickstoff und weitere Kohlenwasserstoffe enthält, durch Absorption in ein geeignetes Absorptionsmittel Propan und Propen, gegebenenfalls mit Restmengen an Stickstoff, in das Absorptionsmittel absorbiert und daraus wieder durch Strippen mit Luft desorbiert. Auf diese Weise werden Wasserstoff, die Kohlenoxide, weiteren Kohlenwasserstoffe sowie Stickstoff entfernt. Anschließend wird der das Propen und gegebenenfalls Propan enthaltende Strom der Oxidationsstufe 2 zugeführt, in der Propen zu Acrolein und/oder Acrylsäure oxidiert wird. Nach der Oxidation 2 wird das erhaltene Produkt der Aufarbeitung 3 zugeführt. In dieser werden die Zielprodukte Acrolein beziehungsweise Acrylsäure abgetrennt. Verbleibendes nicht umgesetztes Propen sowie Propan, Kohlenoxide und gegebenenfalls Reste von Stickstoff und Sauerstoff werden wiederum der Absorptions- und Desorptionsstufe 1 zugeführt.

Bei den weiteren Figuren bezeichnen gleiche Bezugsziffern das Gleiche wie in Fig. 1.

Im Unterschied zu Fig. 1 ist in Fig. 2 eine Propandehydrierung 4 vorgeschaltet, die mit oder ohne Sauerstoffzufuhr durchgeführt werden kann. Das bei der Propandehydrierung erhaltene Gasgemisch, das neben dem Propan und dem Propen Wasserstoff, Kohlenoxide und eventuell Reste an Stickstoff und Kohlenwasserstoffen enthält, wird der Absorptions- und Desorptionsstufe 1 zugeführt.

Im Unterschied zu Fig. 1 liegt in Fig. 3 anstelle der Propenoxidation 2 eine Propanoxidationsstufe 22 vor, bei der Propan zu Acrolein und/oder Acrylsäure oxidiert wird.

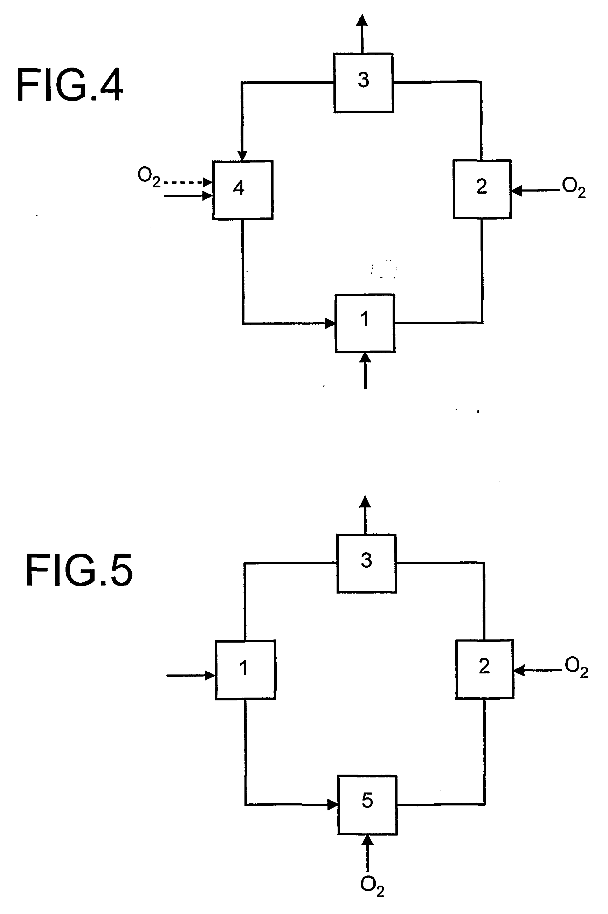

In Fig. 4 wird nach der Aufarbeitungsstufe 3 eine Propandehydrierungstufe 4 mit oder ohne Sauerstoffzufuhr durchgeführt und das in dieser Stufe erhaltene Gasgemisch in die Absorptions- und Desorptionsstufe 1 zurückgeführt.

Fig. 5 zeigt eine weitere bevorzugte Ausgestaltung des Verfahrens, bei dem der Absorptions- und Desorptionsstufe 1 eine Propandehydrierung 5 mit Sauerstoffzufuhr nachgeschaltet ist.

Fig. 6 zeigt ein weiteres bevorzugtes Verfahren. Das Verfahren von Fig. 6 folgt dem Verfahrensschema von Fig. 4. In Fig. 4 liegen in der Propandehydrierungstufe 4 drei Reaktoren vor, von denen im ersten vor der Propandehydrierung (PDH) eine Kohlenmonoxidnachverbrennung (CO-NV) stattfindet, während in den beiden nachfolgenden Reaktoren vor der Propandehydrierung (PDH) eine Wasserstoffnachverbrennung (H2-NV) stattfindet. Diese Nachverbrennungen dienen der Energiezufuhr für die Propandehydrierung. Die Anzahl der Reaktoren in der Propandehydrierung ist nicht auf 3 Reaktoren beschränkt. In die Propandehydrierung 4 wird über Leitung (30) Propan zugeführt. Über Leitung (6) kann Luft zugeführt werden. Das nach der Propandehydrierung erhaltene Gasgemisch wird über einen Wärmetauscher W und einen Verdichter V in Stufe 1 einer Absorptionskolonne Klund einer Desorptionskolonne K2 zugeführt. Das Absorptionsmittel wird nach der Desorption in K2 wieder der Absorptionskolonne K1 zugeführt. Nicht absorbierte Gase werden als Abgas (33), gegebenenfalls über eine Verbrennungsanlage E, aus dem Prozeß geführt. Der das abgetrennte Propan und/oder Propen enthaltende Strom wird der Oxidationsstufe 2 zugeführt, die vorliegend mit vier Oxidationsreaktoren gezeigt ist. Die Anzahl der Oxidationsreaktoren ist jedoch nicht auf diese Anzahl beschränkt. Anschließend wird das Zielprodukt Acrolein und/oder Acrylsäure in Stufe 3 aufgearbeitet und über Leitung (31) abgezogen. Nicht umgesetztes Propan und/oder Propen wird als Kreisgas über Leitung (32) zusammen mit den anderen vorliegend nicht bei der Absorption abgetrennten gasförmigen Bestandteilen der Propandehydrierung 4 rückgeführt.

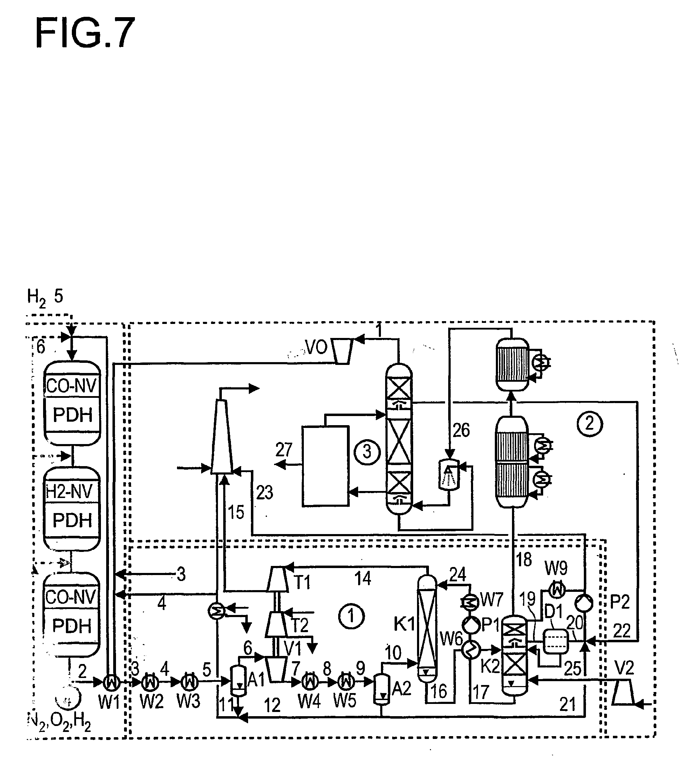

In Fig. 7 wird Kreisgas (1) aus der Aufarbeitungsstufe 3, das bei Temperaturen zwischen 10 bis 90°C und bei Drücken zwischen 0,8 bis 5 bar anfällt und zum Beispiel mit Hilfe eines Verdichters V0 weiter auf Drücke zwischen 2 bis 10 bar verdichtet werden kann, im Gegenstrom in einem Wärmetauscher W1 zum Reaktionsgas (2) aus der Propan-Dehydrierung (PDH) 4 auf Temperaturen zwischen 100 bis 650°C aufgeheizt. Die Druckangabe in bar bezieht sich hier und im folgenden bei Fig. 7 auf den Absolutdruck.

Als Verdichter kommen alle geeigneten, dem Fachmann bekannten Ausführungsformen, die im Folgenden noch näher benannt werden, in Frage.

Der Kreisgasstrom (1) enthält etwa 40 bis 80 Vol% $N_2$, etwa 1 bis 5 Vol% $CO_2$, 0,1 bis 2 Vol% CO, 2 bis 5 Vol% $O_2$, 0,5 bis 25 Vol% $H_2O$, weitere Oxidations-Nebenprodukte und etwa 5 bis 40 Vol% nicht umgesetztes Propan und etwa 0,1 bis 3 Vol% nicht umgesetztes Propen. Vor oder nach der Aufheizung wird dem Gas Frisch-Propan (3) und vorzugsweise Wasser oder Wasserdampf (4) zugemischt, bevor es in die PDH 4 geleitet wird. Als Frisch-Propan kommen alle verfügbaren Propan-haltigen Gase beziehungsweise Flüssigkeiten in Frage. Günstig sind jedoch Propanquellen wie technisches Propan (> 90% Propan-Gehalt, insbesondere >95% Propan-Gehalt, besonders bevorzugt >98% Propangehalt mit geringem $C_4^+$-Anteil) oder Rein-Propan (>99% Propan-Gehalt). Das molare Verhältnis von Wasser beziehungsweise Wasserdampf zu Propan im Gasstrom beträgt zwischen 0,05 bis 5, vorzugsweise aber 0,1 bis 2. Es kann günstig sein, diesem Gasstrom zusätzlich $H_2$ (5), Luft (6) oder ein $O_2$-haltiges Gas sowie weitere in der PDH exotherm umsetzbare Komponenten wie zum Beispiel CO oder CO/$H_2$-Mischungen, wie zum Beispiel Synthesegas, zuzumischen. Die Reinheit dieser Gase ist keiner Begrenzung unterworfen. Die Exothermie der Oxidation der brennbaren Komponenten in der PDH dient zur Deckung der Endothermie der PDH-Reaktion, so dass von außen weniger und im günstigsten Fall keine zusätzliche Wärme zur Deckung der Reaktionsenthalpie für die PDH zugeführt werden muss.

Die PDH wird zwischen 0,3 und 10 bar, vorzugsweise 1 bis 5 bar, und bei Temperaturen zwischen 350 und 700°C, vorzugsweise 400 bis 600°C, betrieben. Als mögliche Reaktoren für die PDH kommen alle dem Fachmann bekannten Ausführungsformen in Frage, zum Beispiel axial durchströmte Apparate wie Hordenreaktoren, aber auch Apparate mit mehreren hohlzylinderFörmig angeordneten Katalysatorschüttungen, die radial durchströmt werden, oder mehrere Einzelapparate, zum Beispiel in Kolonnen-, Torten- oder Kugelform. Die Anzahl der Reaktoren in der PDH ist nicht auf 3 beschränkt.

Vorzugsweise werden mehrere Einzelapparate verwendet, da dadurch die Zwischeneinspeisung von weiteren Gasen einfach möglich wird und außerdem einzelne Katalysatorschüttungen während des Fahrbetriebes getrennt von den anderen besonders behandelt, wie zum Beispiel regeneriert, werden können. Dazu wird zum Beispiel der Reaktor mit der zu regenerierenden Katalysatorschüttung durch geeignete Sperrorgane, wie zum Beispiel Schieber, Ventile oder Klappen, die sich in den Verbindungsleitungen zwischen den Reaktoren befinden, vom Hauptgasstrom getrennt und anschließend die zur Regenerierung notwendigen Gase, zum Beispiel $N_2$, $H_2$, Magerluft oder Luft beziehungsweise $O_2$-reiche Gase, über die Katalysatorschüttung geleitet und Ablagerungen vom Katalysator entfernt. Die restlichen Reaktoren, deren Zahl insgesamt zwischen 1 und 20, vorzugsweise jedoch zwischen 2 und 5 liegen kann, werden weiterhin mit dem Hauptgasstrom beaufschlagt und produzieren hauptsächlich das gewünschte Produkt Propen.

In den PDH-Reaktoren können die Katalysatorschichten auf Rosten, Schaltungen aus Inertmaterial oder ähnlichen dem Fachmann bekannten Auflagevorrichtungen liegen. Der Form des Katalysators sind keine Beschränkungen auferlegt. Formen wie zum Beispiel Splitt, Kugeln, Stränglinge, Ringe, Zylinder oder auch strukturierte Packungen sowie Monolithe sind einsetzbar. Günstig sind solche Geometrien, die einen kleinen Druckverlust aufweisen.

Zur Verteilung des zugeführten Gases auf die Katalysatorschüttung können dem Fachmann bekannte Gasverteiler, wie zum Beispiel Siebböden, Ring- oder Rohrverteiler, sowie regellose Schüttungen oder strukturierte Packungen, wie zum Beispiel statische Mischer, eingesetzt werden.

In den PDH-Reaktoren können mehrere Katalysatorschichten mit unterschiedlichen Funktionen angeordnet sein. Werden mehrere Katalysatorschichten verwendet, so ist es günstig, vor der PDH-Katalysator-Schicht eine oder mehrere Katalysatorschichten anzuordnen, an dem beziehungsweise denen bevorzugt zum Beispiel $H_2$, CO und/oder eine weitere oxidierbare Komponente, die nicht Propen oder Propan ist, oxidiert werden können beziehungsweise kann (CO-NV beziehungsweise $H_2$-NV). Denkbar ist jedoch auch, auf zusätzliche der PDH-Katalysator-Schicht vorgeschaltete Katalysatorschichten zu verzichten, wenn der PDH-Katalysator diese Funktion übernimmt oder Propanverluste durch Oxidation des Propans wirtschaftlich vertretbar sind.

Die PDH-Katalysatoren können mit 100 bis 20000 N1 Propan pro Liter Katalysatorschüttung und Stunde betrieben werden. Bevorzugt liegt der Wert jedoch zwischen 500 und 10000, noch besser zwischen 1000 und 10000. Die Gasbelastung für Katalysatoren, die zum Beispiel vorwiegend CO oder $H_2$ und weniger Propan beziehungsweise Propen oxidieren, liegen üblicherweise zwischen 5000 und 30000 N1 Gas pro Liter Katalysatorschüttung und Stunde.

Der Propan-Umsatz in der PDH liegt zwischen 10 und 60%, bevorzugt zwischen 20 und 50% bei Propen-Selektivitäten zwischen 80 und 99,5%, häufig zwischen 88 und 96%. Der Umsatz der zugeführten Gase CO, $H_2$ beziehungsweise anderen Brenngasen ist günstiger weise vollständig. Der Umsatz an während der PDH gebildetem $H_2$ liegt je nach Propan-Umsatz zwischen 1 und 99%, öfters zwischen 10 und 80% und häufig zwischen 30 und 70%.

Der Reaktionsgas (2) aus der PDH enthält etwa 20 bis 60 Vol% $N_2$, etwa 1 bis 5 Vol% $CO_2$, 0,5 bis 45 Vol% $H_2O$, etwa 5 bis 40 Vol% Propan, etwa 1 bis 20 Vol% Propen, etwa 1 bis 20 Vol% $H_2$ sowie weitere Nebenprodukte zum Beispiel Ethan, Ethen, Methan und $C_4$+.

Das Reaktionsgas (2) aus der PDH fällt bei Temperaturen zwischen 400 und 650°C, günstiger zwischen 450 und 600°C, und Drücken zwischen 0,3 und 10 bar, günstiger zwischen 1 und 5 bar an. Es wird im Gegenstrom zum Kreisgas (1) auf Temperaturen, die mindestens 5°C, besser mindestens 50°C und bevorzugt mindestens 100°C über der Eintrittstemperatur des Kreisgases (1) liegen, abgekühlt. Anschließend wird je nach Austrittstemperatur aus dem Gegenstrom-Wärmetauscher W1 der Gasstrom (3) ein- oder mehrstufig auf etwa 10 bis 60°C weiter abgekühlt. Bei der mehrstufigen Abkühlung kann je nach Temperaturniveau in W2 die Abkühlung durch Dampferzeugung oder durch Luftkühlung und die Abkühlung in W3 sowohl durch Luft-, Wasser- oder Solekühlung erfolgen. Dabei kondensiert je nach Druck, Temperatur und $H_2O$-Anteil in den Gasströmen (3) bis (5) Wasser aus, das im Abscheider A1 aus dem Gasstrom (5) abgeschieden wird. Als Gasabscheider kommen alle dem Fachmann bekannten und dafür geeigneten Ausführungsformen in Frage.

Anschließend wird der abgekühlte und gegebenenfalls teilentwässerte Gasstrom (6) auf Drücke zwischen Austrittsdruck aus dem Abscheider A1 und 50 bar verdichtet. Die Verdichtung kann sowohl einstufig als auch mehrstufig mit und ohne Zwischenkühlung erfolgen. Als Verdichter V1 kommen alle dem Fachmann bekannten und dafür geeigneten Ausführungsformen in Frage, wie zum Beispiel Hub- und Drehkolbenverdichter, Schraubenverdichter, Membranverdichter, Drehschieberverdichter, Turboverdichter, Kreiselverdichter sowie Drehkolben- und Kreiselgebläse, bevorzugt kommen jedoch Turboverdichter oder Kreiselverdichter zum Einsatz. Auswahlkriterien für die Verdichterwahl sind sowohl die Druckerhöhung als auch die Menge an zu verdichtendem Gasstrom. Bei der mehrstufigen Verdichtung mit Zwischenkühlung kondensieren bei der Zwischenkühlung Wasser und gegebenenfalls weitere kondensierbare Komponenten, die nach oder während der Zwischenkühlung wie oben beschrieben aus dem Gasstrom abgetrennt werden können, bevor der Gastrom auf die nächste Verdichterstufe geführt wird. Der auf Enddruck verdichtete Gasstrom (7) kann wie oben beschrieben wieder ein- oder mehrstufig abgekühlt werden, wobei wieder Wasser und gegebenenfalls weitere kondensierbare Stoffe aus den Gasströmen (7) bis (9) abgetrennt werden können.

Der Verdichter V1 kann sowohl über Elektromotoren als auch über Dampf- oder Gasturbinen angetrieben werden. Die Auswahl hängt von den Infrastrukturbedingungen ab. Häufig erweist sich der Antrieb mit Dampfturbinen als der wirtschaftlichste.

Die Summe der kondensierten Ströme, zum Beispiel (11) - nach einer Druckerhöhung - und (12), wird, soweit es zur Deckung des $H_2O$ zu Propanverhältnisses vor Eintritt in die PDH notwendig ist, zur PDH rezirkuliert und der Rest wird ausgeschleust und gegebenenfalls verbrannt. Der Kondensatstrom (13) kann vor der Rezirkulation beziehungsweise Ausschleusung verdampft werden oder einer weiteren Behandlung, zum Beispiel einer Reinigung vor der Rezirkulation unterworfen werden.

Der Gasstrom (10) wird anschließend der Absorptionskolonne K1 zugeführt, in der Propan und/oder Propen aus dem Gasstrom abgetrennt werden. Hierbei wird der Gasstrom (10) mit einem Absorptionsmittel in Kontakt gebracht, das die $C_3$-Fraktion und gegebenenfalls weitere Komponenten aufnimmt. Als Absorptionsmittel kommen alle dem Fachmann bekannten Stoffe in Frage, wobei die bereits beschriebenen Absorptionsmittel bevorzugt eingesetzt werden. Bevorzugt wird der Gasstrom (10) mehrstufig im Gegenstrom zum Absorptionsmittel geführt. Die Absorption kann bei Temperaturen zwischen 10 und 150°C, besser zwischen 20 und 80°C und bevorzugt zwischen 30 und 60°C und bei Drücken zwischen 1 und 50 bar, besser zwischen 3 und 30 bar und bevorzugt zwischen 5 und 20 bar erfolgen.

Als Absorber K1 kommen alle dem Fachmann bekannten Ausführungsformen in Frage, wie zum Beispiel in "Thermische Trennverfahren"; Klaus Sattel, VCH, 1988, (ISBN 3-527-28636-5) beschrieben. Zu bevorzugen sind Kolonnen mit Einbauten. Als Einbauten kommen ebenfalls alle dem Fachmann bekannten Ausführungsformen in Frage, wie zum Beispiel Siebböden, Dualflow-Böden, Glocken-Böden, Tunnel-Böden, Gitter-Böden, Ventil-Böden oder regelose Schüttungen zum Beispiel aus Ringen (zum Beispiel der Firma Raschig), Pall-Ringe, Intalox Sattel, Berl-Sattel, Super-Sattel, Torus-Sattel, Interpack-Füllkörper oder Dtahtgeweberinge sowie strukturierte Packungen (zum Beispiel Sulzer-Kerapak, oder Sulzer-Packung BX oder CY, oder zum Beispiel Packungen der Firma Montz sowie Packungen weiterer Hersteller). Besonders geeignet ist Ralu-Pak 250.YC von der Firma Raschig. Zu bevorzugen sind dabei Einbauten, die eine große Flüssigkeitsbelastung beziehungsweise Berieselungsdichte erlauben, wie zum Beispiel unstrukturierte Schüttungen oder strukturierte Packungen. Die mögliche Berieselungsdichte sollte günstiger weise größer 50, noch besser größer 80 $m^3$ Flüssigkeit pro $m^2$ freie Querschnittsfläche und Stunde sein. Die Einbauten können sowohl metallischer, keramischer Natur aber auch aus Kunststoff sein, oder aus einer Zusammensetzung aus mehreren Werkstoffen bestehen. Wesentlich bei der Wahl des Materials für die Schüttungen und Packungen ist, dass das Absorptionsmittel diese Einbauten gut benetzt.

Das Verhältnis der Ströme zwischen dem der Absorption zugeführten Absorptionsmittel (24) und Gasstrom (10) folgt den thermodynamischen Anforderungen und hängt von der Anzahl der Trennstufen, der Temperatur, dem Druck, den Absorptionseigenschaften des Absorptionsmittels und des geforderten Trenngrades ab. Üblich sind Verhältnisse von 1:1 bis 50:1, insbesondere 1:1 1 bis 30:1, bevorzugt 1:1 1 bis 20:1 in kg/kg bei einer theoretischen Stufenzahl von 1 bis 40, insbesondere 2 bis 30, bevorzugt 5 bis 20. Die Definition einer theoretischen Stufenzahl kann der Fachliteratur, wie zum Beispiel "Thermische Trennverfahren"; Klaus Sattel, VCH, 1988, (ISBN 3-527-28636-5) entnommen werden.

Der von Propan und/oder Propen abgereicherte Gasstrom (14) kann, um gegebenenfalls Verluste an Absorptionsmittel zu reduzieren, einer Quenchstufe zugeführt werden. Die Funktionsweise einer Quenchung wird im folgenden bei der Beschreibung der Desorptionsstufe noch näher erklärt.

Der Gasstrom (14) kann nach Verlassen einer eventuellen Quenchstufe entspannt werden. Die Entspannung kann dabei sowohl einstufig als auch mehrstufig durch Drosselung ohne Energierückgewinnung erfolgen oder ein- oder mehrstufig in einer Gasturbine T1 unter Rückgewinnung von mechanischer Energie. Im Falle der Rückgewinnung von mechanischer Energie ist es unter Umständen notwendig, den Gasstrom (14) vor dem Einleiten in die Turbine aufzuheizen. Die Aufheizung des Gasstromes kann sowohl direkt durch katalytische sowie unkatalytische Oxidation von im Gasstrom enthaltenen oder von außen zugeführten brennbaren und oxidierenden Komponenten erfolgen, sowie durch indirekte Wärmezufuhr mit Hilfe von Dampf oder Außenbefeuerung. Die bei der Entspannung gewonnene mechanische Energie kann sowohl direkt als Mit- oder Hauptantrieb für einen der Verdichter, bevorzugt V1, und/oder zur Erzeugung von Strom genutzt werden.

Nach der Entspannung kann, je nach Reinheit des erhaltenen Abgasstromes (15), dieser einer katalytischen oder unkatalytischen Abgasverbrennung zugeführt oder gleich in die Umgebung abgeführt werden.

Der vorwiegend mit Propan (2 bis 30 Vol%) und/oder Propen (2 bis 30 Vol%) und gegebenenfalls noch weiteren Komponenten (zum Beispiel $CO_2$, $C_2^-$, $C_4^+$, $H_2O$) beladene Absorptionsmittelstrom (16) wird, wenn erforderlich entspannt, und anschließend der Desorptionskolonne K2 zugeführt. Die Entspannung kann dabei sowohl ohne Rückgewinnung der mechanischen Energie ein- oder mehrstufig durch Drosselung als auch unter Rückgewinnung von mechanischer Energie (zum Beispiel in einer Turbine oder rückwärts laufenden Kreiselpumpe) erfolgen. Zudem kann es nützlich sein, den Strom (16) vor der Desorption aufzuwärmen. Diese Aufwärmung erfolgt bevorzugt als GegenstromWärmetausch mit dem Strom (17) in W6. Außerdem kann es nützlich sein, Strom (16) darüber hinaus weiter aufzuheizen.

Die Desorption in K2 von Propan und/oder Propen kann sowohl destillativ, durch einfachen Flash wie auch durch Strippen erfolgen. Die Desorption wird dabei durch die Absenkung des Druckes auf 0,1 bis 10 bar, insbesondere auf 1 bis 5 bar und bevorzugt auf 1,5 bis 3 bar unterstützt.

Erfolgt die Desorption destillativ, so kann dieser Trennschritt nach allen dem Fachmann bekannten Wissen erfolgen. Eine besonders einfache Ausführungsform der Desorption ist das einstufige Flashen beziehungsweise Entspannungsverdampfung des beladenen Lösungsmittels in einem dafür geeignetem Apparat. Es kann sinnvoll sein, den Strom (16) vor dem Flashen auf 20 bis 300°C, insbesondere auf 40 bis 200°, bevorzugt auf 50 bis 150°C aufzuheizen. Der Apparat ist dabei so zu gestalten, dass sowohl die thermodynamische Trennung zwischen Propan beziehungsweise Propen und dem Lösungsmittel gut erfolgt, wie auch die fluiddynamische Trennung zwischen Gas und Flüssigkeit. Der Apparat kann zum Beispiel Zylinderform oder Kugelform, wie auch andere dem Fachmann bekannte Bauformen, aufweisen. Besitzt der Apparat Zylinderform, dann kann dieser Zylinder sowohl stehend als auch liegend angeordnet sein. Der Zulauf zum Flashapparat liegt vertikal gesehen in der Regel zwischen dem Gas- und dem Flüssigkeitsablauf. Der Apparat besitzt im einfachsten Fall keine zusätzlichen Einbauten. Zur besseren thermodynamischen Trennung können im Apparat Einbauten, wie sie dem Fachmann für die Destillation, Absorption und Strippung bekannt sind, eingebaut werden, insbesondere solche, wie sie im Text oben für die Absorption beschrieben wurden. Für die bessere fluiddynamische Trennung, können zusätzlich Einbauten, wie sie dem Fachmann für die Gas/Flüssig-Trennung bekannt sind, wie zum Beispiel Gestricke, Umlenkbleche oder ähnliches in den Flashapparat integriert werden. Zudem kann der Flashapparat Vorrichtungen enthalten, die einen Wärmeeintrag erlauben, wie zum Beispiel beheizte Rohrschlangen oder beheizte Wände.

Steht wie im vorliegenden Fall Luft oder ein ähnliches Strippmedium (zum Beispiel Wasserdampf, $N_2$, Frischpropan oder ein weiteres im Prozess benötigtes Gas) zur Verfügung, wird dieses sinnvollerweise verwendet, um den Flash-Vorgang zu unterstützen.

Eine spezielle Ausführung dafür ist die mehrstufige Strippung der flüchtigen Komponenten Propan und/oder Propen mit dem Ausgangsgasstrom (25) zur Oxidation (selbstverständlich werden dabei wie im einstufigen Fall alle zusätzlich absorbierten Komponenten aus dem Strom (10) sowie eventuell gebildete Stoffe entsprechend ihrer Flüchtigkeit mit gestrippt). Im einfachsten Fall ist der Ausgangsgasstrom (25) die zur Oxidation des Propens zu Acrolein beziehungsweise Acrylsäure benötigte Luft. Die Verdichtung der Luft beziehungsweise des Ausgangsgasstromes kann dabei sowohl vor als auch nach der Desorption erfolgen. Der Ausgangsgasstrom (25) kann aber neben der Luft auch Kreisgas aus dem Acrylsäure-Prozess sowie Wasserdampf, Frisch-Propan oder eine weitere inertisierende gasförmige Komponente enthalten. Besonders günstig ist, wenn während der Desorption der Ausgangsgasstrom (25) im Gegenstrom oder Kreuzstrom zum flüssigen Absorptionsmittel geführt wird. Im Fall des Gegenstromes kann der Desorptions-Apparat beziehungsweise Desorber so ausgeführt sein, wie die im Text oben beschriebene Absorptionskolonne.

Die Kreuzstromführung kann dann sinnvoll sein, wenn während der Desorption der Explosionsbereich durchschritten wird. Das ist dann der Fall, wenn der Ausgangsgasstrom (25) bezüglich der Verbrennungsneigung ein mageres Gasgemisch ist und der mit Propan und/oder Propen beladene Gasstrom (18) nach der Desorption bezüglich der Verbrennungsneigung ein fettes Gasgemisch ist. Als "mager" wird ein Gasgemisch in diesem Zusammenhang dann bezeichnet, wenn der Gehalt an brennbaren Stoffen zu niedrig ist, um entzündbar zu sein und als "fett" wird ein Gasgemisch in diesem Zusammenhang dann bezeichnet, wenn der Gehalt an brennbaren Stoffen zu hoch ist, um entzündbar zu sein.

Im Fall der Kreuzstromführung wird der gesamte Ausgangsgasstrom nicht in den Sumpf sondern in Teilströmen aufgeteilt an mehreren geeigneten Stellen entlang der Desorptionskolonne eingebracht und zwar in der Art, dass an keiner Stelle im Desorptionsapparat ein entzündbares Gasgemisch vorliegt. Die Desorptionskolonne kann dabei stehend wie auch liegend angeordnet sein.

Eine weitere Möglichkeit der Umgehung der Explosionsproblematik bei der Desorption von brennbaren Komponenten mit $O_2$-haltigen Gasströmen ist das Mischen des Ausgangsgasstromes vor Eintritt in die Desorptionskolonne mit einem Stoff (zum Beispiel Propan, Propen, Methan, Ethan, Butan, $H_2O$, etc.) in der Art, dass das Ausgangsgasgemisch vor Eintritt in die Desorptionskolonne selbst schon "fett" ist. Es ist aber auch denkbar, den Ausgangsgasstrom zu splitten und in den Sumpf der Desorptionskolonne ein Propan beziehungsweise Propen-freies Ausgangsgas einzuleiten, um eine möglichst gute Abreicherung des Absorptionsmittelstromes (17) von Propan und/oder Propen zu erreichen und ein zum Beispiel mit Propan und/oder Propen "angefettetes" Ausgangsgas in den Bereich der Desorptionskolonne einzuleiten, in dem ein zündbares Gasgemisch entstehen kann.

Der von Propan und/oder Propen abgereicherte Absorptionsmittelstrom (17) kann nach einem eventuellen Gegenstromwärmetausch (W6) und einer Druckerhöhung mit Hilfe einer Pumpe (P1) ein oder mehrstufig (zum Beispiel in W7) weiter abgekühlt werden und über Leitung (24) wieder dem Absorber K1 zugeführt werden.

Generell kann die mehrstufige Desorption bei allen Drücken und Temperaturen stattfinden. Vorteilhaft sind jedoch Drücke, die kleiner und Temperaturen, die höher sind als in der Absorption. Im vorliegenden Fall sind Drücke zwischen 1 und 5 bar, insbesondere zwischen 2 und 3 bar und Temperaturen zwischen 20 und 200°C, insbesondere 30 und 100°C, besonders bevorzugt 35 und 70°C anzustreben.

Das Verhältnis der Ströme zwischen Absorptionsmittel (17) und Ausgangsgas (25) folgt den thermodynamischen Anforderungen und hängt von der Anzahl der Trennstufen, der Temperatur, dem Druck und den Desorptionseigenschaften des Absorptionsmittels und des geforderten Trenngrades ab. Üblich sind Verhältnisse von 1:1 bis 50:1, insbesondere 5:1 bis 40:1, bevorzugt 10:1 bis 30:1 in kg/kg bei einer theoretischen Stufenzahl von 1 bis 20, insbesondere 2 bis 15, bevorzugt 3 bis 10.

Generell wird der mit Propen oder Propan und Propen beladene Ausgangsgasstrom ohne weitere Behandlung den Oxidationsstufen 2 zugeführt. Es kann jedoch gemäß einer nicht erfindungsgemäßen ausführungsform sinnvoll sein, den Ausgangsgasstrom vor der Oxidation einer weiteren Verfahrensstufe zuzuführen, um zum Beispiel die Verluste an mitgestripptem Absorptionsmittel zu reduzieren. Die Abtrennung des Absorptionsmittels aus dem beladenen Ausgangsgasstrom zur Oxidation kann nach allen dem Fachmann bekannten Verfahrensschritten erfolgen. Eine mögliche Ausführungsform ist das Quenchen des beladenen Ausgangsgasstromes mit Wasser. In diesem Fall wird das Absorptionsmittel aus dem beladenen Ausgangsgasstrom mit Wasser herausgewaschen. Diese Wäsche beziehungsweise das Quenchen kann im Kopf der Desorptionskolonne über einem Flüssigkeits-Fangboden oder in einem eigenen Apparat erfolgen. Zur Unterstützung des Trenneffektes können im Quench-Apparat Einbauten installiert werden, wie sie der Fachmann für die Destillation, Absorption und Desorption kennt und wie sie im Text oben für die Absorption beschrieben wurden. Das gleiche gilt für den Quench-Apparat, insofern er als eigener Apparat gestaltet wird. Nachdem das Wasser das Absorptionsmittel aus dem mit Propen oder Propan und Propen beladenen Ausgangsgasstrom herausgewaschen hat, kann das Wasser/Absorptionsmittel-Gemisch (19) einer Phasentrennung D1 zugeführt und der behandelte Ausgangsgasstrom (18) nach einer eventuellen Vorwärmung in die Propen-Oxidationsstufe 2 geführt werden.

Die Phasentrennung kann in allen dem Fachmann bekannten Ausführungsformen, wie sie zum Beispiel auch in der Flüssig/Flüssig-Extraktion zum Einsatz kommen, ausgeführt werden. Im einfachsten Fall sind das liegende oder stehende langgestreckte Apparate mit und ohne Einbauten, in denen sich die organische Absorptionsmittel-Phase vom QuenchWasser trennt. Das Durchmesser zu Längen-Verhältnis kann hier zwischen 1 zu 1 bis 100, insbesondere 1 zu 1 bis 10, bevorzugt 1 zu 1 bis 3 liegen. Der Apparat kann geflutet oder mit einem Gaspolster betrieben werden. Zur besseren Abtrennung der organischen Absorptionsmittelphase kann der Apparat mit einem Dom ausgerüstet sein, aus dem die organische Phase abgezogen werden kann. Zur Unterstützung der Phasentrennung können alle dem Fachmann dafür bekannten Einbauten wie zum Beispiel Gestricke, Wickelkerzen oder Umlenkbleche eingebaut sein. Selbstverständlich können auch rotierende Phasenscheider, wie zum Beispiel Zentrifugen eingesetzt werden.

Nach der Phasentrennung kann das abgetrennte Absorptionsmittel (20) wieder der Desorption zugeführt werden. Das Quenchwasser kann vor Wiedereintritt in den Quench-Apparat gegebenenfalls einer Kühlung oder Aufwärmung in einem Wärmetauscher (W9) zugeführt werden. Günstiger weise werden große Wassermengen mit Hilfe einer Pumpe (P2) umgepumpt. In Betracht kommen Berieselungsdichten im Quench-Apparat von größer 30, insbesondere größer 50 und bevorzugt größer 80 aber kleiner 1000, insbesondere 500 und bevorzugt kleiner 300 m$^3$ Wasser pro m$^2$ freie Querschnittsfläche des Quenchapparates und Stunde.

Die Wasser-Verluste beim Quenchen können durch Kondensat (21) aber auch durch Sauerwasser (22) aus dem Acrylsäure-Herstellutigsverfahren gedeckt werden. Um Aufpegelungen zu vermeiden, kann ein Teil des umlaufenden Quenchwassers als Reinigungs(*Purge*)-Strom (23) ausgeschleust und der Verbrennungsanlage beziehungsweise einer anderen entsorgenden Behandlung (zum Beispiel in einer Kläranlage) zugeführt werden.

Besitzt der Gasstrom (18) für die Propen-Oxidation 2 eine Temperatur < 90°C, insbesondere < 70°C bei Drücken von 1 bis 3 bar, so kann es sinnvoll sein, diesem Strom noch zusätzlich Wasser zuzuführen. Dies kann durch Zumischen von Wasserdampf erfolgen oder durch Sättigung des Stromes (18) in einem Wassersättiger, in einer Art, wie sie dem Fachmann bekannt sind.

Der so behandelte Gasstrom besitzt eine Zusammensetzung von 30 bis 70 Vol% N$_2$, 5 bis 20 Vol% O$_2$, 2 bis 15 Vol% Propen, 2 bis 40 Vol% Propan, 0,5 bis 25 Vol% H$_2$O, und enthält weitere Komponenten wie zum Beispiel CO$_2$, Methan, Ethan, Ethen, C$_4$$^+$. Er kann der Oxidation 2 zugeführt werden, die wie oben bechrieben oder aus der Patentliteratur bekannt durchgeführt werden kann. Dabei kann die Propen- beziehungsweise Acrolein-Oxidation in Salzbadreaktoren zum Beispiel der Deggendorfer-Werft nach dem Stand der Technik oder in anderen Reaktor-Typen durchgeführt werden. Zwischen der 1.Oxidationsstufe zu Acrolein und der 2.Oxidationsstufe zu Acrylsäure kann noch einmal eine Lufteinspeisung oder Wasserdampfeinspeisung stattfinden oder nicht. Der höhere C$_3$-Gehalt im Gas (18) zur Oxidation 2 fördert in jedem Fall die Auskopplung der Reaktionswärme aus dem Reaktionsraum. Propenbelastungen von 50 bis 350, insbesondere 90 bis 300, bevorzugt 100 bis 250 N1 Propen pro Liter Katalysatorschüttung und Stunde sind geeignet.

Die Abtrennung der Acrylsäure aus dem Reaktionsgas (26) der Oxidation 2 in Stufe 3 kann wie oben beschrieben mit beispielsweise einem hochsiedenden Lösungsmittel, wie zum Beispiel einem Gemisch aus Diphyl und Dimethylphthalat, oder durch Absorption in Wasser sowie durch fraktionierte Kondensation erfolgen.

Die Reinigung der Acrylsäure kann durch Strippung und Destillation oder durch Azeotropdestillation oder durch Kristallisation erfolgen.

Das in Fig. 7 beschriebene Verfahren eignet sich sowohl für die Nachrüstung aller bestehender Anlagen zur Herstellung von Acrolein und/oder Acrylsäure als auch in Kombination mit neuen Acrylsäureanlagen.

Überraschenderweise wurde festgestellt, dass trotz in der Regel zu erwartender Reste an Absorptionsmittel im Gas B keine Probleme bei der Oxidation beziehungsweise dem Oxidationskatalysator auftreten. Es konnten auch keine Probleme mit irgendwelchen Oxidationsprodukten, die sich während der Oxidation gegebenenfalls aus dem Absorptionsmittel bilden, beobachtet werden.

Es war deshalb überraschend, dass bei dem erfindungsgemäßen Verfahren eine Absorption eingesetzt werden kann. Im Gegensatz zu der in der japanischen Schrift JP-A-10 36311 eingesetzten Adsorption ist die vorliegend für Propen oder Propan und Propen eingesetzte Absorption wesentlich leichter und wirtschaftlicher handzuhaben.

Weiterhin bietet die vorliegende Erfindung den Vorteil, daß bestehende, von Propen ausgehende Anlagen zur Herstellung von Acrolein und/oder Acrylsäure in günstiger Weise auf das kostengünstigere Propan als Ausgangsmaterial umgerüstet werden können.

**Vergleichs-Beispiel**

Es wird Acrylsäure nach einem Verfahren hergestellt, wie es in Fig. 7 dargestellt ist. Die im folgenden verwendeten Bezugsziffern beziehen sich deshalb auf Fig. 7.

2090 Nl/h des Kreisgases (1) aus der Aufarbeitungsstufe 3, das bei einer Temperatur von 30°C und einem Druck von 1,2 bar anfällt, werden mit Hilfe eines Verdichters V0 auf 2,0 bar verdichtet und im Gegenstrom in einem Wärmetauscher W1 zum Reaktionsgas (2) aus der Propan-Dehydrierung (PDH) auf eine Temperatur von 450°C aufgeheizt. Die Druckangabe in bar bezieht sich hier und im folgenden in diesem Beispiel auf den Absolutdruck.

Der Kreisgasstrom (1) enthält 60,3 Vol.-% N$_2$, 1,2 Vol.-% CO$_2$, 0,5 Vol.-% CO, 3,4 Vol.-% O$_2$, 1,9 VoL-% H$_2$O, 32,2 Vol.-% Propan und 0,4 Vol.-% Propen sowie weitere Oxidations-Nebenprodukte. Vor der Aufheizung werden dem Kreisgasstrom 170 Nl/h Frisch-Propan (3) und Wasserdampf (4) zugemischt, bevor es in die PDH geleitet wird. Als Frisch-Propan wird technisches Propan (>98% Propangehalt mit 100 Gew.-ppm C$_4$$^+$-Anteil) eingesetzt. Das molare Verhältnis von Wasserdampf zu Propan im Gasstrom beträgt 0,5.

Das Gasgemisch wird dem 1. Reaktor von 4 Reaktoren zugeführt. Der Innendurchmesser der Reaktoren beträgt 50 mm. Die Reaktoren sind konstruktiv so gestaltet, dass sie autotherm betrieben werden können. Jeder Reaktor enthält eine 110 mm hohe KatalysatorSchüttung aus Stränglingen (D = 3 mm, L = 5mm).

In den 4 Reaktoren wird Propan zu 20% bei einer Propen-Selektivität von 92% umgesetzt.

Das Reaktionsgas (2) aus der PDH enthält 44,9 Vol.-% N$_2$, 2,7 Vol.-% CO$_2$, 16,9 Vol.-% H$_2$O, 24,0 Vol.-% Propan, 5,8 Vol.-% Propen, 5,5 Vol.-% H$_2$ sowie geringe Mengen an weiteren Nebenprodukten, zum Beispiel Ethan, Ethen, Methan und C$_4$+.

Das Reaktionsgas (2) aus der PDH fällt bei 520°C und 1,5 bar an und wird auf 30°C abgekühlt. Dabei kondensieren im Mittel stündlich etwa 350 g Wasser (11) aus.

Anschließend wird der abgekühlte Gasstrom (6) einstufig in einem Kolbenverdichter auf 7,5 bar verdichtet und wieder auf 30 °C abgekühlt. Das dabei auskondensierte Wasser (12) wird zusammen mit dem Kondensat (11) vereint und zum teil wieder verdampft und dem Kreisgasstrom (1) zugemischt. Ein weiterer Teil (Strom (21)) wird in den Quench zugeführt. Der Rest wird ausgeschleust.

2340 Nl/h des Gasstromes (10) werden anschließend in den Sumpf der Absorptionskolonne K1 (Metallmantel, Innendurchmesser = 80 mm, Kolonnenlänge 3 m) geleitet. 60 % des Volumens der Absorptionskolonne sind mit Pakkungselementen von der Firma Montz (Montz-Pak Typ B1) gefüllt.

Der Gasstrom (10) enthält 53,9 Vol.-% Stickstoff, 3,3 Vol.-% Kohlendioxid, 0,4 Vol.-% Wasser, 28,8 Vol.-% Propan, 7,0 Vol.-% Propen, 6,6 Vol.-% Wasserstoff sowie geringe Mengen an weiteren Nebenprodukten wie zum Beispiel Ethan, Ethen, Methan und $C_{4+}$.

Auf den Kopf der Absorptionskolonne K1 werden 35 kg/h $C_3$-armes Tetradecan (24) aus der Desorptionskolonne mit 30 °C geleitet.

Im Abgasstrom (14) sind noch 1150 Vol.-ppm Propan und 750 Vol.-ppm Propen enthalten. Der Abgasstrom (14) aus der Absorptionskolonne wird über ein Druckhalteventil auf Umgebungstemperatur beziehungsweise Umgebungsdruck entspannt und anschließend verbrannt.

Aus dem Sumpf der Kolonne K1 wird der beladene Absorptionsmittelstrom (16) entnommen, auf 2,4 bar über ein Druckhalteventil entspannt und auf den Kopf der Desorptionskolonne K2 geführt.

Die Desorptionskolonne K2 besitzt die gleichen Abmessungen wie die Absorptionskolonne K1 und ist in gleicher Weise mit Füllkörpern bestückt.

In den Sumpf der Desorptionskolonne werden 1310 N1 Druckluft bei 2,45 bar mit 30 °C eingeleitet. Die Desorptionskolonne wird auf 40°C thermostatisiert.

Das Abgas aus der Desorptionskolonne (2190 Nl/h) enthält 30,7 Vol.-% Propan, 7,4 Vol.-% Propen, 12,3 Vol.% $O_2$, 46,4 Vol.-% $N_2$, 1,5 Vol.-% $H_2O$ und 1,6 Vol.-% $CO_2$ und geringe Reste an Tetradecan und wird in einen Quenchapparat, der sich im Kopf der Desorptionskolonne K2 befindet, geleitet.

Der Sumpfablauf der Desorptionskolonne K2 wird über die Pumpe P1 und den Wärmetauscher W7 auf den Kopf der Absorptionskolonne K1 gefördert.

Der Quenchapparat ist eine Metallkolonne mit ebenfalls 80 mm Innendurchmesser und ist in der gleichen Art wie die in der Absorptionskolonne K1 mit Einbauten bestückt. Der Wasserquench wird bei 30 °C betrieben. Im Quenchapparat werden etwa 1201 Wasser pro Stunde auf die Schüttung gesprüht. Aus dem Sumpf des Quench-Apparates wird ein zweiphasiges Flüssigkeitsgemisch entnommen und in einen Phasenscheider geleitet. Der Phasenscheider ist ein liegender Behälter mit 200 mm Durchmesser und 500 mm Länge und enthält ein feines Drahtgestrick, das im ersten Drittel des Phasenscheiders in Strömungsrichtung angeordnet eingebaut ist. Die aus dem Phasenscheider D1 abgezogene wäßrige Phase wird wieder auf den Kopf des Quenchapparates gepumpt. Im Mittel werden stündlich etwa 1 g Tetradecan aus dem Phasenscheider abgezogen und in die Tetradecan-Vorlage geleitet. Die Wasser-Verluste beim Quenchen werden durch Kondensatwasser (21) ausgeglichen.

Der Abgasstrom aus dem Wasser-Quench wird auf 200°C aufgewärmt, bevor er der zweistufigen Oxidation zugeführt wird.

Die Oxidation findet in Modellrohren mit einem Innendurchmesser von 26 mm und einer Länge von 4 m statt. Das erste Modellrohr ist mit 2,7 m eines Katalysators gefüllt, wie er in der EP-A-0 575 879 beschrieben ist, und das zweite Modellrohr ist mit 3m eines Katalysators gefüllt, wie er in der EP-A-0 017 000 beschrieben ist. Zwischen der ersten und zweiten Oxidationsstufe werden stündlich noch zusätzlich 315 N1 Frischluft geleitet.

Die Abtrennung der Acrylsäure aus dem Reaktionsgas (26) der Oxidation und die Reinigung derselben erfolgt wie in der EP-A-0 982 289 beschrieben.

Nach diesem Verfahren werden im Mittel stündlich 440 g Roh-Acrylsäure (27) mit > 99,5 % Acrylsäure gewonnen.

**Patentansprüche**

1. Verfahren zur Herstellung von Acrolein und/oder Acrylsäure aus Propan, wobei das Verfahren folgende Schritte umfasst:

    (a) homogene und/oder heterogen katalytische Dehydrierung von Propan zu Propen unter Erhalt eines Propen oder eines Propen und Propan enthaltenden Gasgemisches A,
    (b) Abtrennung von Propen oder Propen und Propan aus dem Gasgemisch A durch Absorption in ein Absorptionsmittel,
    (c) Abtrennung des Propens oder des Propens und Propans von dem Absorptionsmittel unter Erhalt eines Propen oder eines Propen und Propan enthaltenden Gases B und

(d) Verwendung des in Stufe (c) erhaltenen Gases B für eine Oxidation von Propen zu Acrolein und/oder Acrylsäure,

wobei zwischen Schritt (c) und (d) keine heterogen katalysierte Dehydrierung von Propan ohne Zufuhr von Sauerstoff durchgeführt wird,
wobei die Dehydrierung oxidativ erfolgt und Schritt (d) direkt nach Schritt (c) durchgeführt wird.

2.  Verfahren nach Anspruch 1, **dadurch gekennzeichnet, dass** in Schritt (b) das Gasgemisch A neben dem Propen oder dem Propen und Propan wenigstens eine weitere Komponente ausgewählt aus Wasserstoff, Stickstoff oder Kohlenoxiden enthält.

3.  Verfahren nach Anspruch 1 oder 2, **dadurch gekennzeichnet, dass** in Schritt (b) als Absorptionsmittel wenigstens ein $C_8$-$C_{20}$-Alkan oder -Alken verwendet wird.

4.  Verfahren nach einem der Ansprüche 1 bis 3, **dadurch gekennzeichnet, dass** die Abtrennung des Propens oder des Propens und Propans von dem Absorptionsmittel in Schritt (c) durch Strippung, Entspannungsverdampfung und/oder Destillation erfolgt.

5.  Verfahren nach einem der vorhergehenden Ansprüche, **dadurch gekennzeichnet, dass** die Propandehydrierung in Schritt (a) unter Sauerstoffzufuhr durchgeführt wird.

6.  Verfahren nach einem der vorhergehenden Ansprüche, **dadurch gekennzeichnet, dass** nach Durchführung von Schritt (d) nicht umgesetztes Propan und gegebenenfalls Propen in Schritt (a) zurückgeführt wird.

**Claims**

1.  A process for the preparation of acrolein and/or acrylic acid from propane, the process comprising the following steps:

    (a) homogeneous and/or heterogeneous catalytic dehydrogenation of propane to propene to give a propene- or propene- and propane-comprising gas mixture A,
    (b) separation of propene or propene and propane from gas mixture A by absorption in an absorbent,
    (c) separation of the propene or the propene and propane from the absorbent to give a propene-or propene- and propane-comprising gas B and
    (d) use of the gas B obtained in stage (c) for an oxidation of propene to acrolein and/or acrylic acid,

    no heterogeneously catalyzed dehydrogenation of propane without a supply of oxygen being carried out between steps (c) and (d).
    the dehydrogenation being carried out oxidatively and step (d) taking place directly after step (c).

2.  The process according to claim 1, wherein, in step (b), the gas mixture A, in addition to the propene or the propene and propane, comprises at least one further component selected from hydrogen, nitrogen and oxides of carbon.

3.  The process according to claim 1 or 2, wherein at least one $C_8$-$C_{20}$-alkane or $C_8$-$C_{20}$-alkene is used as the absorbent in step (b).

4.  The process according to any of claims 1 to 3, wherein the separation of the propene or the propene and propane from the absorbent in step (c) is carried out by stripping, flashing and/or distillation.

5.  The process according to any of the preceding claims, wherein the propane dehydrogenation in step (a) is carried out with a supply of oxygen.

6.  The process according to any of the preceding claims, wherein, after step (d) has been carried out, unconverted propane and, if appropriate, propene is recycled to step (a).

**Revendications**

1. Procédé de fabrication d'acroléine et/ou d'acide acrylique à partir de propane, le procédé comprenant les étapes suivantes :

   (a) la déshydrogénation sous catalyse homogène et/ou hétérogène de propane en propène pour obtenir un mélange gazeux A contenant du propène ou du propène et du propane,
   (b) la séparation du propène ou du propène et du propane du mélange gazeux A par absorption dans un milieu absorbant,
   (c) la séparation du propène ou du propène et du propane du milieu absorbant pour obtenir un gaz B contenant du propène ou du propène et du propane; et
   (d) l'utilisation du gaz B obtenu à l'étape (c) pour une oxydation du propène en acroléine et/ou acide acrylique,

   dans lequel on n'effectue pas de déshydrogénation sous catalyse hétérogène de propane entre les étapes (c) et (d) sans apport d'oxygène,
   dans lequel la déshydrogénation s'effectue par oxydation et l'étape (d) est effectuée directement après l'étape (c).

2. Procédé selon la revendication 1, **caractérisé en ce qu'**à l'étape (b), le mélange gazeux A contient, en plus du propène ou en plus du propène et du propane, au moins un autre composant choisi parmi l'hydrogène, l'azote ou des oxydes de carbone.

3. Procédé selon la revendication 1 ou 2, **caractérisé en ce qu'**à l'étape (b), on utilise comme milieu absorbant au moins un alcane ou un alcène en $C_8$-$C_{20}$.

4. Procédé selon l'une quelconque des revendications 1 à 3, **caractérisé en ce que** la séparation du propène ou du propène et du propane du milieu absorbant à l'étape (c) s'effectue par strippage, vaporisation éclair et/ou distillation.

5. Procédé selon l'une quelconque des revendications précédentes, **caractérisé en ce que** la déshydrogénation du propane de l'étape (a) s'effectue par apport d'oxygène.

6. Procédé selon l'une quelconque des revendications précédentes, **caractérisé en ce qu'**après réalisation de l'étape (d), on recycle à l'étape (a) le propane qui n'a pas réagi et éventuellement le propène.

FIG.1

FIG.2

FIG.3

FIG.4

FIG.5

# FIG.6

# FIG.7